# EUROPEAN PATENT SPECIFICATION

(11) **EP 3 728 561 B1**
(45) Date of publication and mention of the grant of the patent: **18.05.2022**
(21) Application number: 18826368.5
(22) Date of filing: 21.12.2018
(51) Int. Cl.: C12M 3/06, C12N 5/071, C12N 5/00

(54) **NOVEL MULTI-ORGAN-CHIPS ESTABLISHING DIFFERENTIATION OF IPSC-DERIVED CELLS INTO ORGAN EQUIVALENTS**
NEUARTIGE MULTI-ORGAN-CHIPS, DIE DIFFERENZIERUNG VON IPSC-ABGELEITETEN ZELLEN IN ORGANÄQUIVALENTEN AUFBAUEN
NOUVELLES PUCES MULTI-ORGANES ÉTABLISSANT UNE DIFFÉRENCIATION DE CELLULES ISSUES DE CELLULES SOUCHES PLURIPOTENTES INDUITES (IPSC) EN ÉQUIVALENTS D'ORGANES

(30) Priority: 22.12.2017 EP 17210375; 22.12.2017 US 201762609664 P
(43) Date of publication of application: 28.10.2020
(73) Proprietor: TissUse GmbH, 13347 Berlin (DE)
(72) Inventor: MARX, Uwe, 15528 Spreenhagen (DE); RAMME, Anja, 14052 Berlin (DE)
(74) Representative: Grünecker Patent- und Rechtsanwälte PartG mbB
(86) International application number: PCT/EP2018/086483
(87) International publication number: WO 2019/122291

(56) References cited:
- WO-A1-2014/052835
- WO-A2-2017/136462
- ILKA MASCHMEYER ET AL: "A four-organ-chip for interconnected long-term co-culture of human intestine, liver, skin and kidney equivalents", LAB ON A CHIP, vol. 15, no. 12, 1 January 2015 (2015-01-01), pages 2688-2699, XP055459923, ISSN: 1473-0197, DOI: 10.1039/C5LC00392J
- MATERNE EVA-MARIA ET AL: "A multi-organ chip co-culture of neurospheres and liver equivalents for long-term substance testing", JOURNAL OF BIOTECHNOLOGY, ELSEVIER, AMSTERDAM, NL, vol. 205, 9 February 2015 (2015-02-09), pages 36-46, XP029240260, ISSN: 0168-1656, DOI: 10.1016/J.JBIOTEC.2015.02.002
- YUKI IMURA ET AL: "Micro Total Bioassay System for Ingested Substances: Assessment of Intestinal Absorption, Hepatic Metabolism, and Bioactivity", ANALYTICAL CHEMISTRY, vol. 82, no. 24, 15 December 2010 (2010-12-15), pages 9983-9988, XP055163485, ISSN: 0003-2700, DOI: 10.1021/ac100806x
- MASCHMEYER ILKA ET AL: "Chip-based human liver-intestine and liver-skin co-cultures - A first step toward systemic repeated dose substance tes", EUROPEAN JOURNAL OF PHARMACEUTICS AND BIOPHARMACEUTICS, ELSEVIER SCIENCE PUBLISHERS B.V., AMSTERDAM, NL, vol. 95, 6 April 2015 (2015-04-06), pages 77-87, XP029282584, ISSN: 0939-6411, DOI: 10.1016/J.EJPB.2015.03.002
- KYTTALA AIJA ET AL: "Genetic Variability Overrides the Impact of Parental Cell Type and Determines iPSC Differentiation Potential", STEM CELL REPORTS, vol. 6, no. 2, February 2016 (2016-02), pages 200-212, XP002779239,
- GIOVANNI G GIOBBE ET AL: "Functional differentiation of human pluripotent stem cells on a chip", NATURE METHODS, vol. 12, no. 7, 1 June 2015 (2015-06-01), pages 637-640, XP055328192, ISSN: 1548-7091, DOI: 10.1038/nmeth.3411
- FRANK SONNTAG ET AL: "Design and prototyping of a chip-based multi-micro-organoid culture system for substance testing, predictive to human (substance) exposure", JOURNAL OF BIOTECHNOLOGY, vol. 148, no. 1, 1 July 2010 (2010-07-01), pages 70-75, XP055185845, ISSN: 0168-1656, DOI: 10.1016/j.jbiotec.2010.02.001
- AMOGH SIVARAPATNA ET AL: "Arterial specification of endothelial cells derived from human induced pluripotent stem cells in a biomimetic flow bioreactor", BIOMATERIALS., vol. 53, 1 June 2015 (2015-06-01), pages 621-633, XP055270220, GB ISSN: 0142-9612, DOI: 10.1016/j.biomaterials.2015.02.121
- YOSUKE K. KUROKAWA ET AL: "Human Induced Pluripotent Stem Cell-Derived Endothelial Cells for Three-Dimensional Microphysiological Systems", TISSUE ENGINEERING. PART C, METHODS DEC 2008, vol. 23, no. 8, 1 August 2017 (2017-08-01) , pages 474-484, XP055485163, US ISSN: 1937-3384, DOI: 10.1089/ten.tec.2017.0133
- OBI S ET AL: "Shear stress induces arterial differentiation of bone marrow-derived endothelial progenitor cells", MICRO-NANOMECHATRONICS AND HUMAN SCIENCE, 2009. MHS 2009. INTERNATIONAL SYMPOSIUM ON, IEEE, PISCATAWAY, NJ, USA, 9 November 2009 (2009-11-09), pages 650-655, XP031579353, ISBN: 978-1-4244-5094-7
- R. BOOTH ET AL: "A multiple-channel, multiple-assay platform for characterization of full-range shear stress effects on vascular endothelial cells", LAB ON A CHIP, vol. 14, no. 11, 1 January 2014 (2014-01-01), pages 1880-1890, XP055485066, ISSN: 1473-0197, DOI: 10.1039/C3LC51304A
- JACKSON G. DESTEFANO ET AL: "Effect of shear stress on iPSC-derived human brain microvascular endothelial cells (dhBMECs)", FLUIDS AND BARRIERS OF THE CNS, vol. 14, no. 1, 4 August 2017 (2017-08-04) , XP055485049, DOI: 10.1186/s12987-017-0068-z
- JONATHAN SHEMESH ET AL: "Flow-induced stress on adherent cells in microfluidic devices", LAB ON A CHIP, vol. 15, no. 21, 1 January 2015 (2015-01-01), pages 4114-4127, XP055485067, ISSN: 1473-0197, DOI: 10.1039/C5LC00633C

## Description

### TECHNICAL FIELD

The present disclosure relates to novel multi-organ-chips establishing the differentiation of induced pluripotent stem cell (iPSC)-derived cells into organ equivalents on microfluidic devices and corresponding methods of generating organ equivalents. The present disclosure also relates to novel bioengineered tissue constructs mimicking organ barriers generated with iPSC-derived endothelial cells and/or organoids bioprinted in, and/or seeded on, a hydrogel. The present disclosure further relates to methods of bioengineering organ constructs comprising co-culturing iPSC-derived organ precursor cells and iPSC-derived fibroblasts and endothelial cells. The present disclosure specifically relates to a microfluidic device comprising: (i) iPSC-derived hepatocyte precursor cells; (ii) iPSC-derived intestinal precursor cells; (iii) iPSC-derived renal tubular precursor cells; and (iv) iPSC-derived neuronal precursor cells; wherein the iPSC-derived precursor cells according to (i), (ii), (iii) and (iv) are differentiated from a single donor iPSC reprogrammed from a single type of somatic cell.

### TECHNICAL BACKGROUND

Microfluidic devices are capable of emulating human biology *in vitro* at the smallest biologically acceptable scale. In contrast to conventional (static) cell and tissue cultures, microphysiological systems apply dynamic fluid flow for physiological nutrition of the tissues as a factor for mimicking organ function with a minimum use of human cells and tissues. The technology of multi-organ-chips is of particular interest to the pharmaceutical industry in terms of drug development and toxicity testing. Multi organ-on-a-chip models can enhance the fundamental understanding of complex biological processes and are considered to bring about more rapid, accurate, cost-effective, and clinically relevant testing of drugs as well as cosmetics. There is thus a growing recognition in the art that *in vitro* testing using microphysiological systems implemented on microfluidic devices can be more effective and also be more reliable than *in vivo* testing.

Stem cells have the ability to continuously divide and are capable of differentiating into various kinds of cells. In recent years, stem cell-based approaches for functional organ generation have emerged, in particular to address the shortage of organs for transplantation, and the culturing of stem cells on organ-on-a-chip devices has been reported in the art. In particular, Giobbe et al. (Nat. Methods, 2015, 12(7), 637-640) describe the functional differentiation of pluripotent stem cells into cardiomyocytes and hepatocytes on a chip, and Zhang et al. (Future Sci. OA, 2017, 3(2), FSO197) describe mesenchymal stem cell cultures and differentiation in microfluidic devices towards organ-on-a-chip. However, there are no reports towards the co-culturing of different stem cell-derived organ precursor cells to establish multi-organ-chips with a number of models emulating organ functionality, in particular no reports about the co-culturing of different induced pluripotent stem cell (iPSC)-derived cells for generating organ equivalents on microfluidic devices.

Maschmeyer et al. (Lab on a Chip, 2015, 15(12), 2688-2699) relates to a four-organ chip for interconnected long-term co-culture of human intestine, liver, skin and kidney equivalents.

WO 2014/052835 A1 relates to a chip comprising different types of cells derived from intestine, liver, kidney, or blood-brain barrier (BBB) tissues.

Materne et al. (Journal of Biotechnology, 2015, 205(24), 36-46) relates to a multi-organ chip co-culture of neurospheres and liver equivalents for long-term substance testing.

Imura et al. (Analytical Chemistry, 2010, 82(24), 9983-9988) relates to a micro total bioassay system for ingested substances and the assessment of intestinal absorption, hepatic metabolism and bioactivity.

Maschmeyer et al. (European Journal of Pharmaceutics and Biopharmaceutics, 2015(95), 77-87) relates to chip-based human liver-intestine and liver-skin co-cultures.

Kyttälä et al. (Stem Cell Reports, 2016, 6(2), 200-212) relates to the comparison of transcriptomic, epigenetic, and differentiation propensities of genetically matched human iPSCs derived from fibroblasts and blood and describes that iPSC lines derived from the same donor are highly similar to each other.

WO 2017/136462 A2 relates to a combination of cell culture systems and microfluidic fluidic systems, in particular to methods of culturing gastrointestinal cells derived from iPSCs on microfluidic chips.

There is a need in the art for multi-organ microfluidic devices establishing the differentiation of induced pluripotent stem cell (iPSC)-derived cells into organ equivalents and corresponding methods of generating organ equivalents. The present disclosure addresses this need in particular by providing novel microfluidic devices comprising: (i) iPSC-derived hepatocyte precursor cells; (ii) iPSC-derived intestinal precursor cells; (iii) iPSC-derived renal tubular precursor cells; and (iv) iPSC-derived neuronal precursor cells; wherein the iPSC-derived precursor cells according to (i), (ii), (iii) and (iv) are differentiated from a single donor iPSC reprogrammed from a single type of somatic cell, and by providing novel methods of bioengineering organ constructs comprising co-culturing iPSC-derived organ precursor cells and iPSC-derived fibroblasts and endothelial cells.

### SUMMARY OF THE INVENTION

The present disclosure provides, *inter alia*, novel multi-organ-chips establishing the differentiation of induced pluripotent stem cell (iPSC)-derived cells into organ equivalents. In particular, the present disclosure relates to a novel microfluidic device for co-culturing different iPSC-derived organ precursor cells to generate multi-organ-chips with a number of models emulating organ functionality. The present disclosure further relates to methods of bioengineering organ constructs comprising co-culturing iPSC-derived organ precursor cells and iPSC-derived fibroblasts and endothelial cells. The present disclosure also relates to novel bioengineered tissue constructs mimicking organ barriers generated with iPSC-derived primary endothelial cells (including, *e.g.*, HDMECs) and/or organoids in or on a hydrogel.

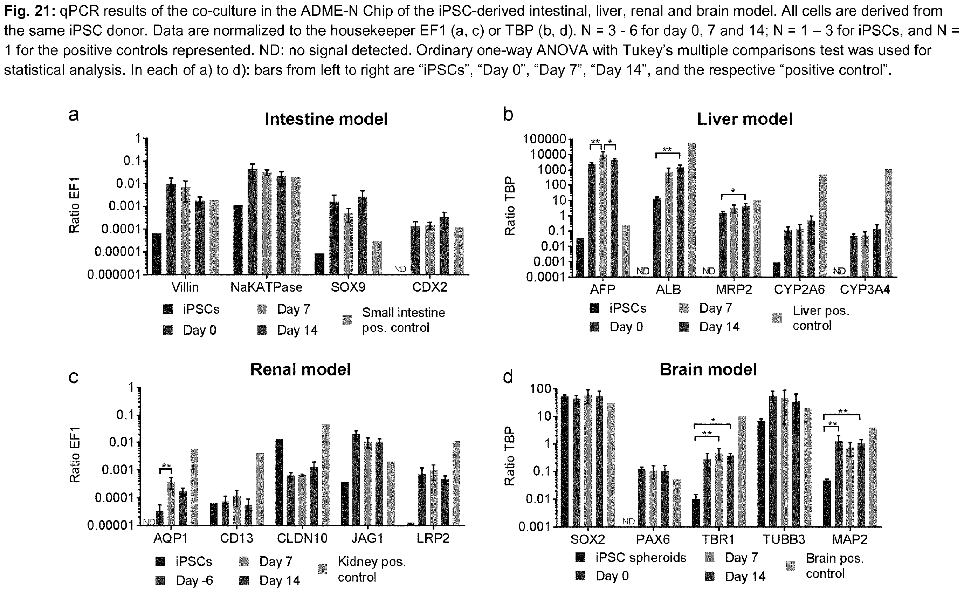

The present disclosure further relates to for implementing a blood circuit and a urine circuit in a microfluidic chip, and establishes a microphysiological system that supports physiological conditions for maintaining stable microenvironments in a system mimicking the interaction and cross-talk of a number of models emulating organ functionality. Surprisingly, it has been found that a four-organ chip with miniaturized human intestine, liver, brain and kidney equivalents, all of them pre-differentiated from iPSCs from the same donor, shows further differentiation of different iPSC-derived organ models over a 14-day cultivation period in a culture medium deprived of growth factors. This further differentiation is attributed to a cross-talk between the organ equivalents. Furthermore, an advanced maturation can be observed for different tissues over time. Significantly, it has been shown that different iPSC-derived organ models are stable during co-cultivation in the ADME-N (= ADME chip plus neuronal equivalent), *i.e.*, maintain their phenotype, and show a defined and consistent marker gene expression towards the directed tissue over time (Experiment 7 and Fig. 21). The present disclosure provides novel microfluidic devices that are suitable for assay establishment for pharmacokinetic-pharmacodynamic analysis in the context of ADME(T) profiling. Specifically, the present disclosure establishes a successful systemic long-term co-cultivation of four different autologous iPSC-derived organ models from a single donor cultured in a physiologically based pharmacokinetic (PBPK) compliant microphysiological system (MPS), without the addition of extra growth factors.

The present invention is defined by the appended claims. In particular, the present invention provides: >
[1] A microfluidic device comprising: (a) cell culture compartments, in which (i) induced pluripotent stem cell (iPSC)-derived hepatocyte precursor cells; (ii) iPSC-derived intestinal precursor cells; (iii) iPSC-derived renal tubular precursor cells; and (iv) iPSC-derived neuronal precursor cells, are separately deposited; wherein the iPSC-derived precursor cells according to (i), (ii), (iii), and (iv) are obtained from a single donor of iPSC obtained from a single type of somatic cell; and (b) further comprising a reservoir compartment, which serves to add nutrient solution to the system.
[2] A microfluidic device comprising: (a) cell culture compartments, in which (i) induced pluripotent stem cell (iPSC)-derived hepatocyte organoids; (ii) iPSC-derived intestinal organoids; and (iii) iPSC-derived renal tubular organoids; are separately deposited; wherein the iPSC-derived organoids according to (i), (ii), and (iii) are obtained from a single donor of iPSC obtained from a single type of somatic cell; and (b) further comprising a reservoir compartment, which serves to add nutrient solution to the system.
[3] The microfluidic device of any one of [1]-[2], wherein the microfluidic device comprises a first circuit and a second circuit, and wherein the cell culture compartments containing the iPSC-derived precursor cells according to (i), (ii), (iii), and (iv), or the iPSC-derived organoids according to (i), (ii), and (iii), respectively, form part of the first circuit, and wherein the second circuit comprising a filtration unit and a reabsorption unit, wherein both circuits are connected with each other via the filtration unit and the reabsorption unit.
[4] The microfluidic device of [3], wherein the filtration unit comprises a filtration barrier that selectively allows the passage of molecules from the first circuit to the second circuit based on size and charge of the molecules.
[5] Use of the microfluidic device of any one of [1]-[4] in analytical testing, diagnostics, research, target validation, toxicity studies, tissue engineering, tissue manufacturing, drug screening, and/or pharmacokinetic-pharmacodynamic analysis.
[6] A method of detecting an analyte in a microphysiological system making use of the microfluidic device of any one of [1]-[4].

In various aspects of the present disclosure, including any microfluidic devices, tissue constructs, methods and uses disclosed herein, the iPSC-derived precursor cells and/or iPSC-derived organoids are cultured with a medium/nutrient solution that does not contain extra growth factors for the iPSC-derived precursor cells and/or iPSC-derived organoids. For example, if the medium contains human adult serum, it does not contain extra growth factors specifically for the individual iPSC-derived precursor cells and/or iPSC-derived organoids, *i.e.*, does not contain, *e.g.*, hepatocyte growth factors for hepatocytes. Accordingly, in various aspects, the medium/nutrient solution does not contain growth factors that are specific for any of the iPSC-derived precursor cells and/or iPSC-derived organoids disclosed herein. The present disclosure encompasses the use of iPSC-derived precursor cells and/or iPSC-derived organoids in any microfluidic devices, tissue constructs, methods and uses disclosed herein, wherein iPSC-derived precursor cells and/or iPSC-derived organoids are co-cultured over a period of at least two weeks. Such a co-culture also encompasses the co-cultivation of four different organoids/organ equivalents. The four different organoids/organ equivalents may be a liver organoid/equivalent, a kidney (or renal) organoid/equivalent, a brain organoid/equivalent, and an intestine organoid/equivalent. In such co-cultures, the culture medium/nutrient solution preferably does not contain growth factors for the iPSC-derived precursor cells and/or iPSC-derived organoids.

Any iPSC-derived organoid described herein may be assembled from/together with iPSC-derived fibroblasts and/or iPSC-derived endothelial cells, wherein the iPSC-derived fibroblasts and/or iPSC-derived endothelial cells are obtained from the same single donor of iPSC (obtained from a single type of somatic cell) as the iPSC-derived organoid. More specifically, the intestinal organoid may be assembled from/together with iPSC-derived fibroblasts and iPSC-derived endothelial cells, *i.e.*, the intestinal organoids may be co-cultured with iPSC-derived fibroblasts and iPSC-derived endothelial cells. Furthermore, the liver organoid may be assembled from/together with iPSC-derived hepatocytes with iPSC-derived fibroblasts. Also, the renal organoid may be assembled from/together with (iPSC-derived) fibroblasts and iPSC-derived endothelial cells. Likewise, the brain organoid may be assembled from/together with iPSC-derived fibroblasts and iPSC-derived endothelial cells. In accordance with the above, the iPSC-derived fibroblasts and/or iPSC-derived endothelial cells may be obtained from the same single donor of iPSC (obtained from a single type of somatic cell) as the iPSC-derived organoid.

### BRIEF DESCRIPTION OF THE DRAWINGS

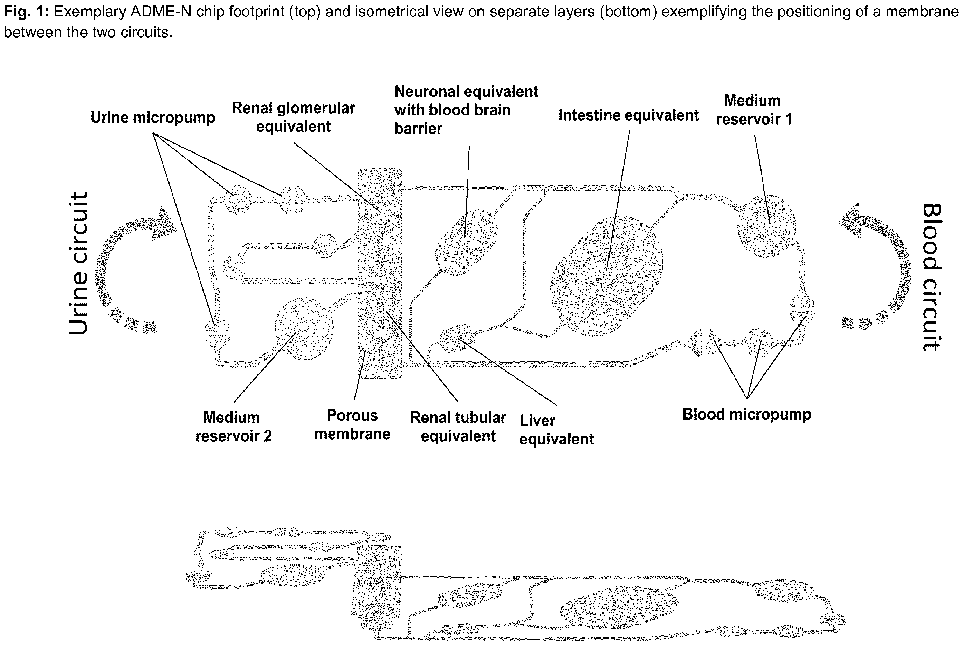

- Figure 1:: shows an exemplary ADME-N chip footprint (top) and isometrical view on separate layers (bottom). The isometrical view exemplifies the positioning of a membrane between the two circuits (blood circuit and urine circuit). ADME-N = ADME chip plus neuronal equivalent.

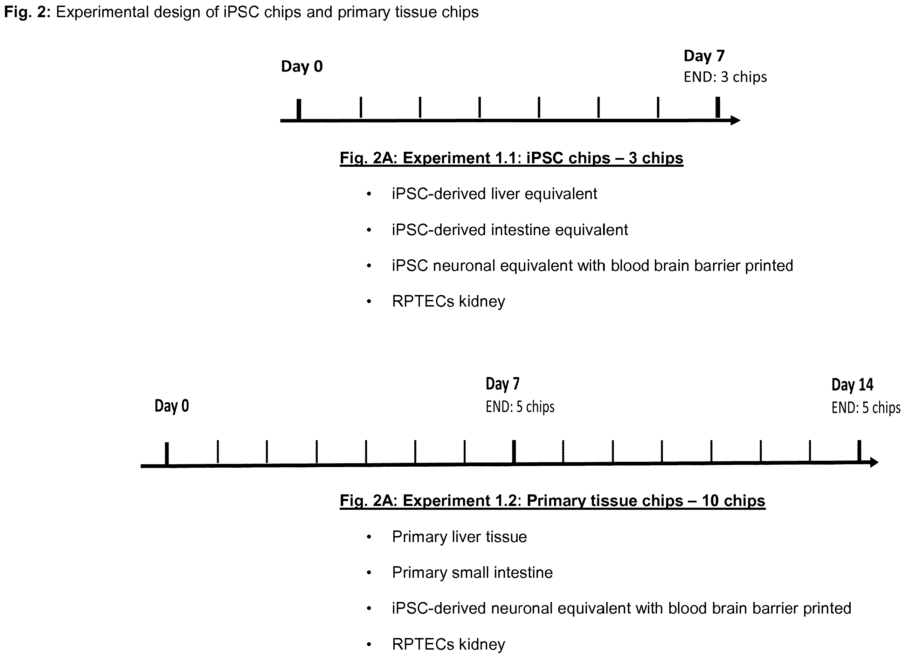

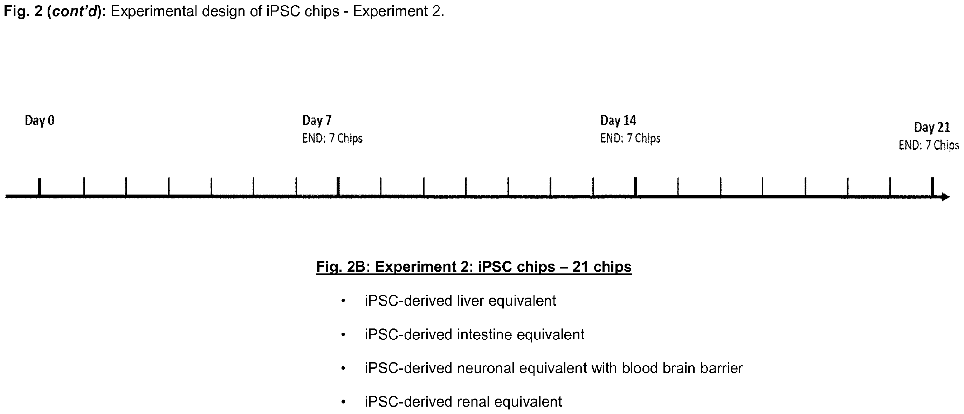

- Figure 2:: shows the experimental design of iPSC chips and primary tissue chips. RPTECs = renal proximal tubule epithelial cell line. iPSC = induced pluripotent stem cell. **2A**: Design of Experiments 1.1 and 1.2, **2B**: Design of Experiment 2.

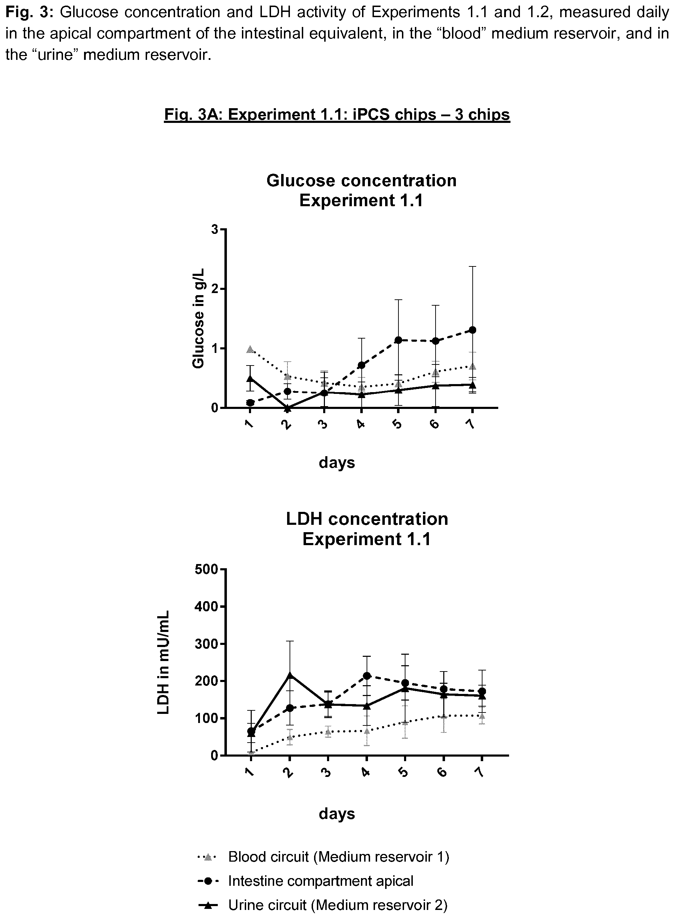

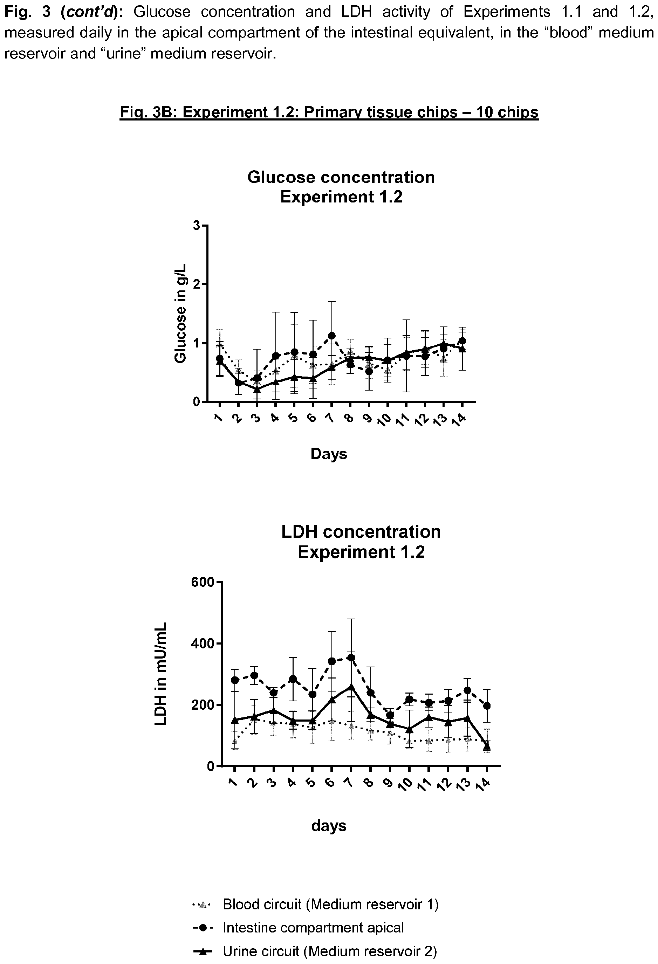

- Figure 3:: shows measurements of glucose concentration and LDH activity. **3A**: measurements of glucose concentration and LDH activity of Experiment 1.1 (iPSC chips); **3B**: measurements of glucose concentration and LDH activity of Experiment 1.2 (Primary tissue chips). Measurements were made daily in the apical compartment of the intestinal equivalent, in the "blood" medium reservoir (for the blood circuit), and in the "urine" medium reservoir (for the urine circuit).

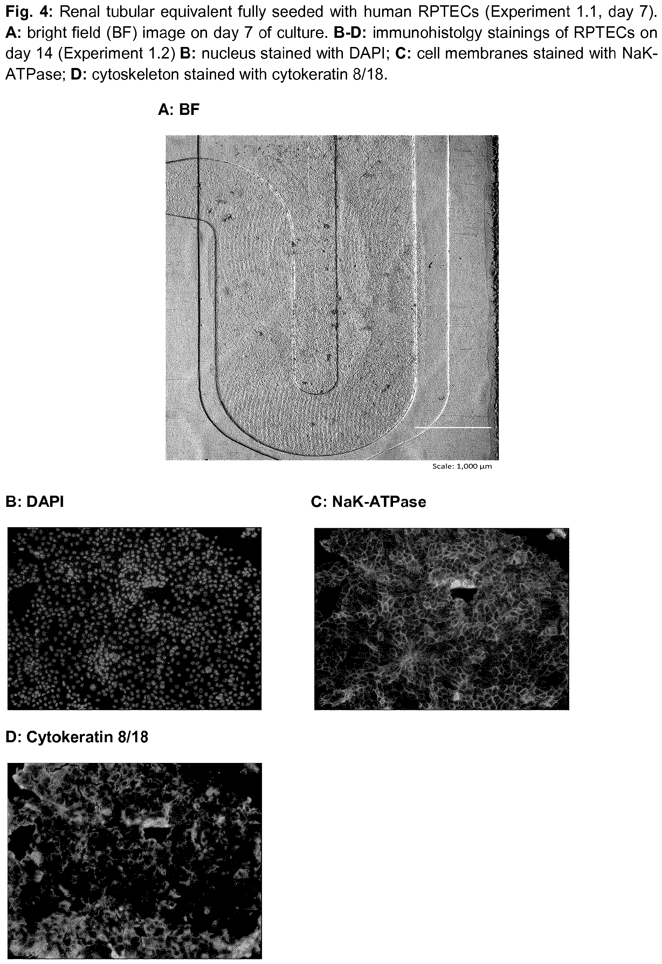

- Figure 4:: shows the renal tubular equivalent of the ADME-N chip fully seeded with human RPTECs (Experiment 1.1, day 7). **A**: bright field (BF) image on day 7 of culture. **B-D**: immunohistologic staining of RPTECs on day 14 (Experiment 1.2) **B**: nucleus stained with DAPI (4',6-Diamidin-2-phenylindol); **C**: cell membranes stained with NaK-ATPase; **D**: cytoskeleton stained with cytokeratin 8/18. RPTECs = renal proximal tubule epithelial cells.

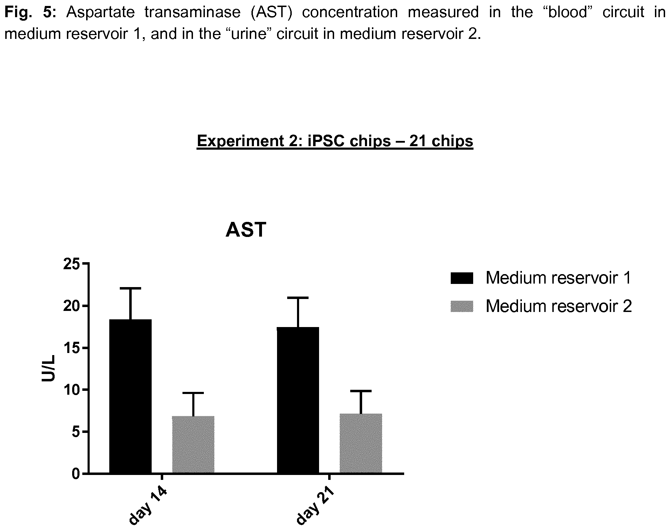

- Figure 5:: shows measurements of aspartate transaminase (AST) concentration in the "blood" circuit in medium reservoir 1, and the "urine" circuit in medium reservoir 2.

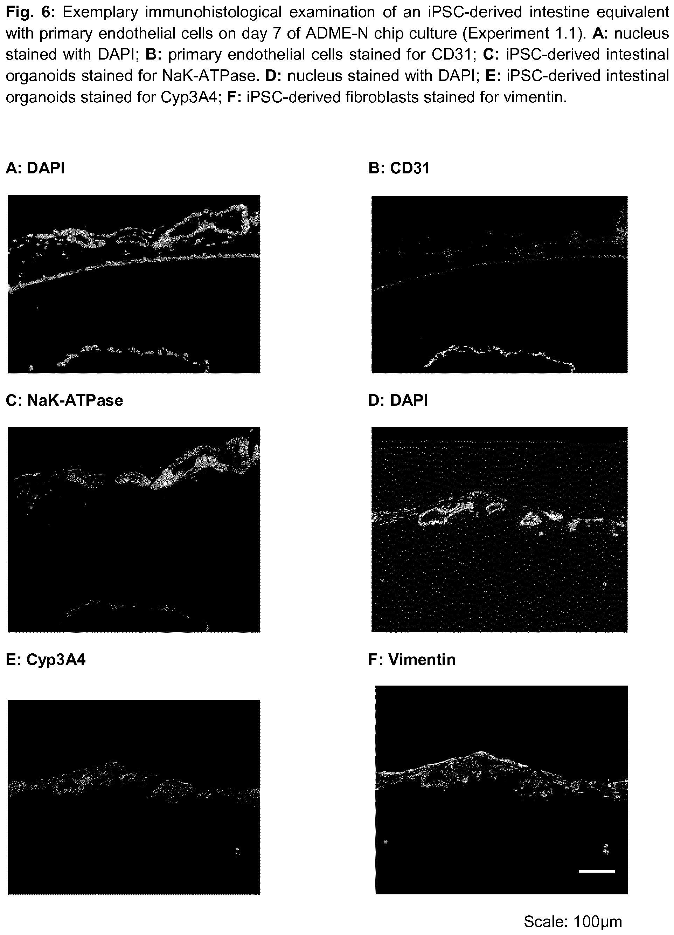

- Figure 6:: shows exemplary immunohistological examination of an iPSC-derived intestine equivalent with primary endothelial cells on day 7 of the ADME-N chip culture (Experiment 1.1). **A**: nucleus stained with DAPI; **B**: primary endothelial cells stained for CD31; **C**: iPSC-derived intestinal organoids stained for NaK-ATPase. **D**: nucleus stained with DAPI; **E**: iPSC-derived intestinal organoids stained for Cyp3A4; **F**: iPSC-derived fibroblasts stained for vimentin.

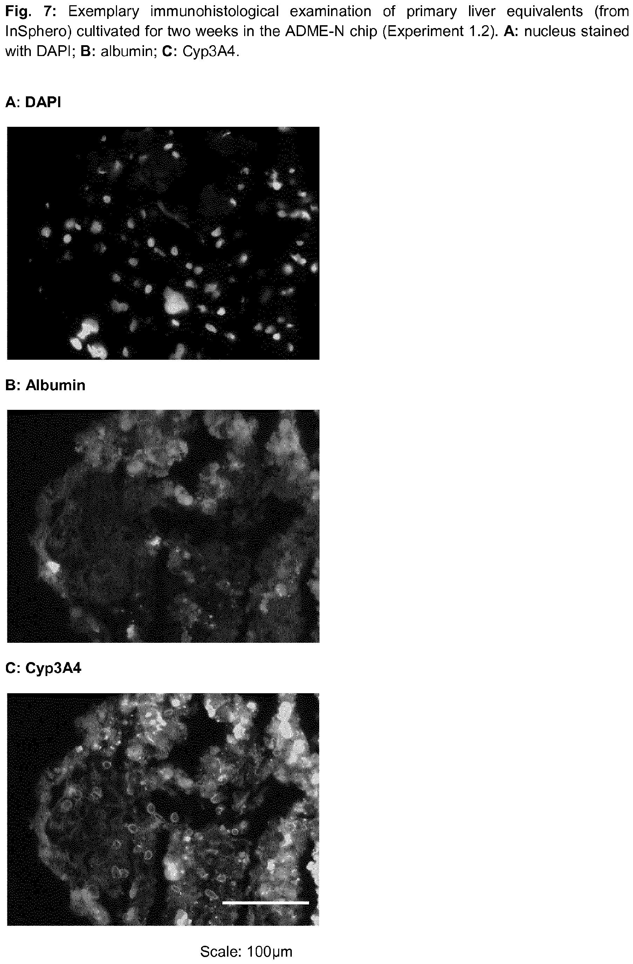

- Figure 7:: shows exemplary immunohistological examination of primary liver equivalents (from InSphero) cultivated for two weeks in the ADME-N chip (Experiment 1.2). **A**: nucleus stained with DAPI; **B**: albumin; **C**: Cyp3A4.

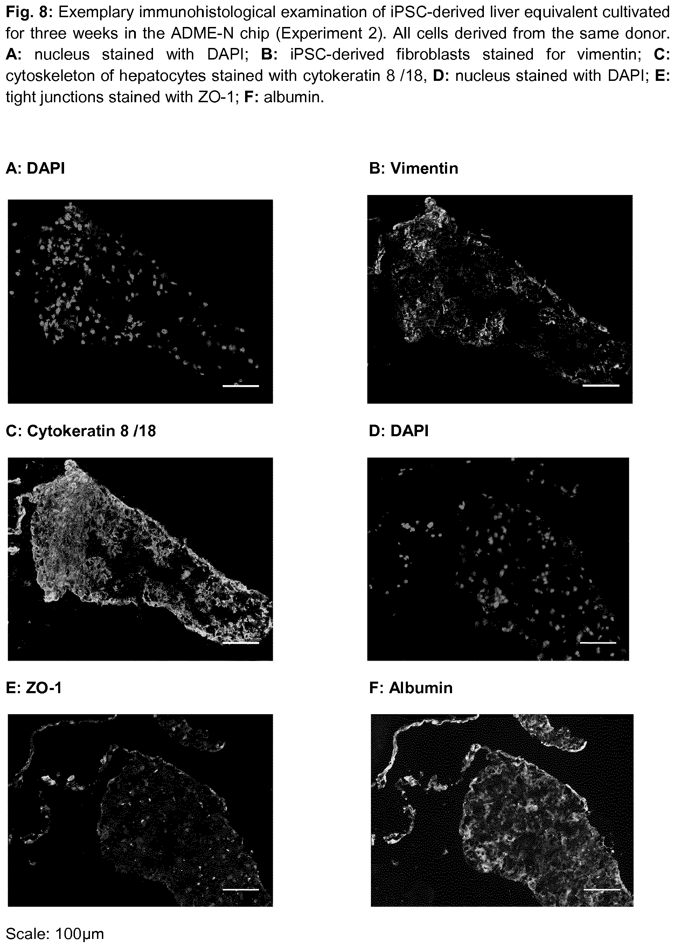

- Figure 8:: shows exemplary immunohistological examination of iPSC-derived liver equivalent cultivated for three weeks in the ADME-N chip (Experiment 2). All cells are derived from the same donor. **A**: nucleus stained with DAPI; **B**: iPSC-derived fibroblasts stained for vimentin; **C**: cytoskeleton of hepatocytes stained with cytokeratin 8 /18, **D**: nucleus stained with DAPI; **E**: tight junctions stained with ZO-1; **F**: albumin.

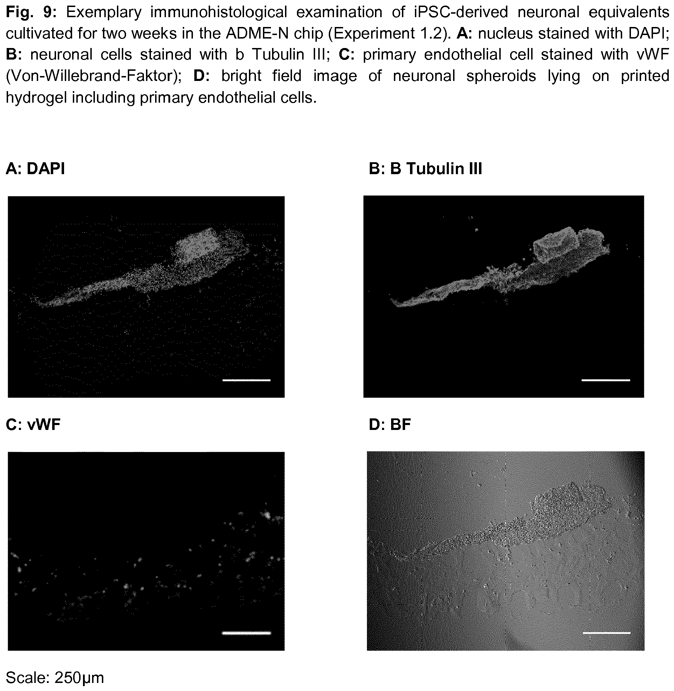

- Figure 9:: shows exemplary immunohistological examination of iPSC-derived neuronal equivalents cultivated for two weeks in the ADME-N chip (Experiment 1.2). **A**: nucleus stained with DAPI; **B**: neuronal cells stained with b Tubulin III; **C**: primary endothelial cell stained with vWF (von-Willebrand-Faktor); **D**: bright field image of neuronal spheroids lying on printed hydrogel including primary endothelial cells.

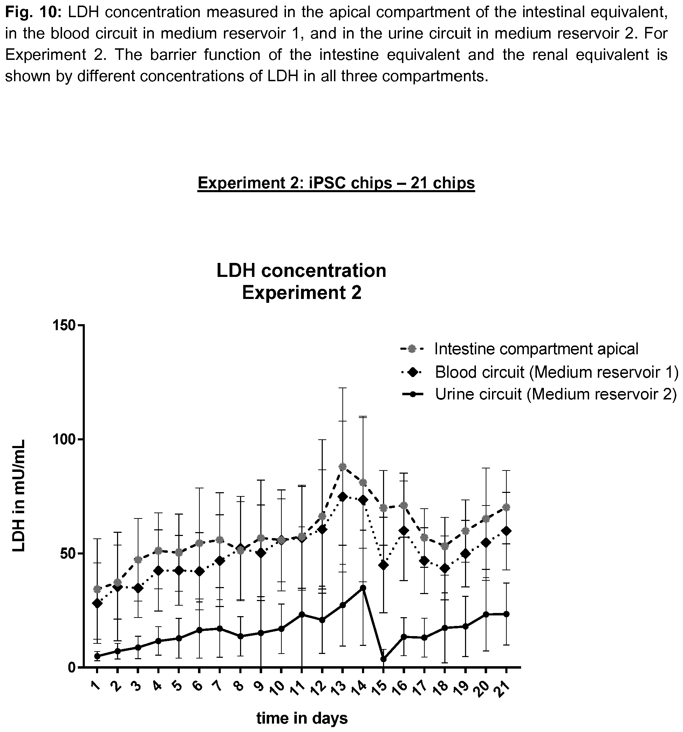

- Figure 10:: shows measurement of LDH concentration in the apical compartment of the intestinal equivalent, in the blood circuit in medium reservoir 1, and in the urine circuit in medium reservoir 2 (Experiment 2). The barrier function of the intestine equivalent and the renal equivalent is shown by different concentrations of LDH in all three compartments.

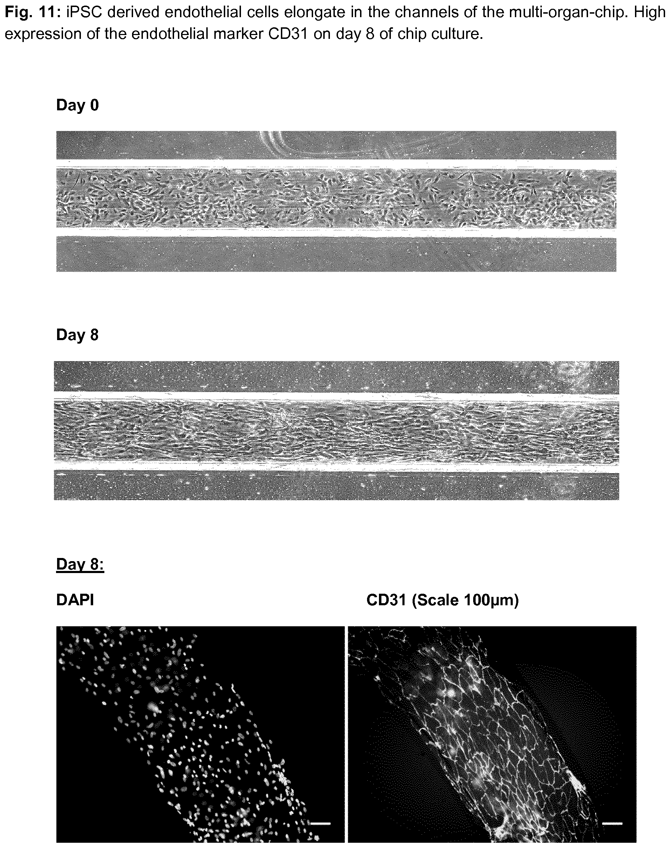

- Figure 11:: shows elongation of iPSC-derived endothelial cells in the channels of the multi-organ-chip. High expression of the endothelial marker CD31 on day 8 of chip culture.

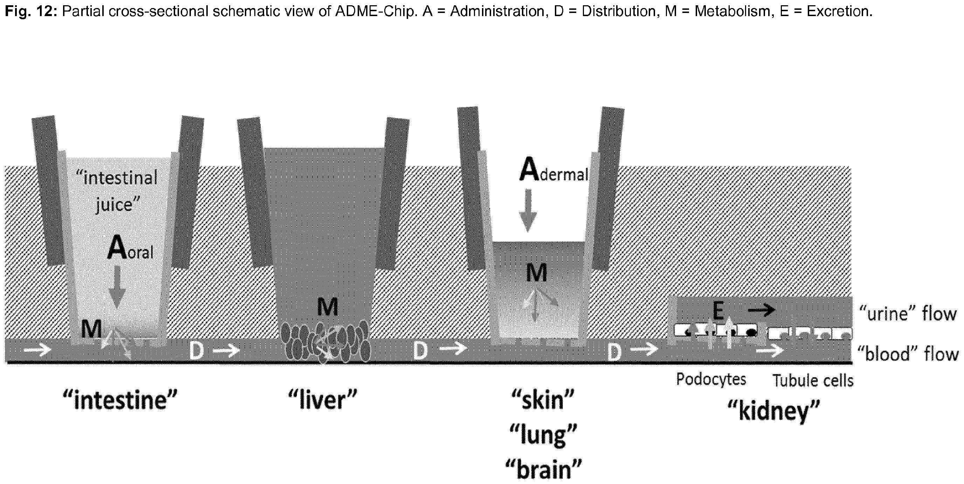

- Figure 12:: shows a cross-sectional schematic view of an ADME-Chip of the present disclosure. A = Administration, D = Distribution, M = Metabolism, E = Excretion.

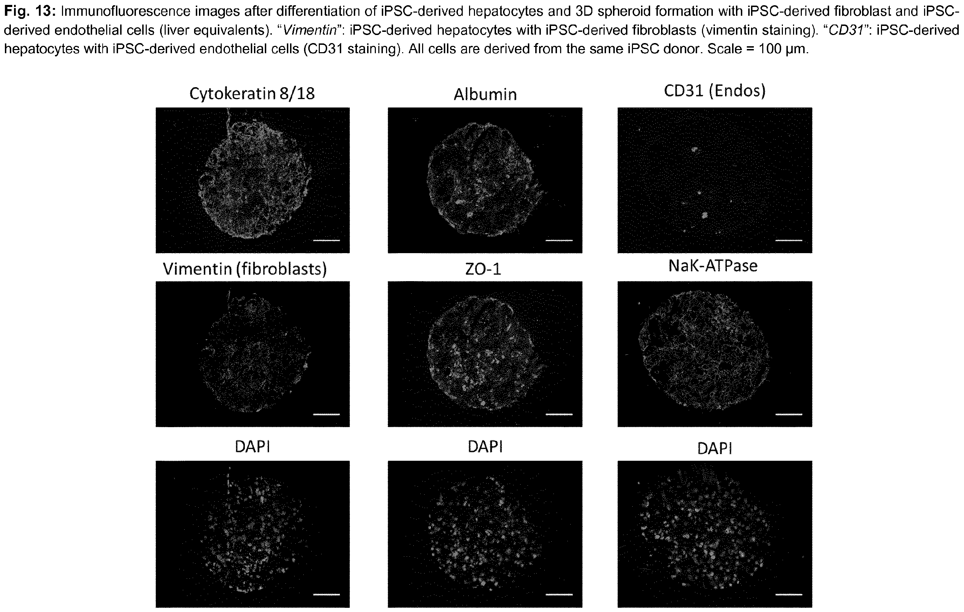

- Figure 13:: shows immunofluorescence images after differentiation of iPSC-derived hepatocytes and 3D spheroid formation with iPSC-derived fibroblast and iPSC-derived endothelial cells (liver equivalents). *Vimentin*: iPSC-derived hepatocytes with iPSC-derived fibroblasts (vimentin staining). CD31: iPSC-derived hepatocytes with iPSC-derived endothelial cells (CD31 staining). All cells are derived from the same iPSC donor. Scale = 100 µm.

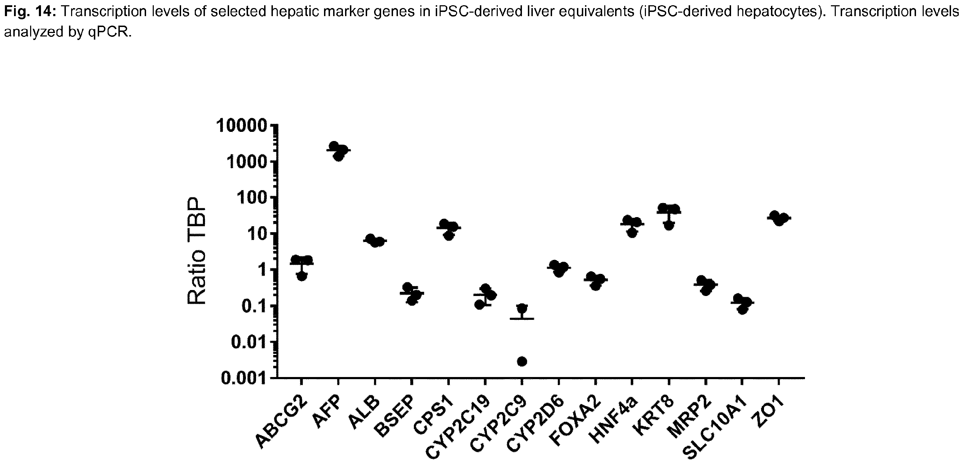

- Figure 14:: shows transcription levels of selected hepatic marker genes in iPSC-derived liver equivalents (iPSC-derived hepatocytes). Transcription levels analyzed by qPCR. The iPSC-derived liver equivalents were cultivated over two days to generate 3D liver spheroids including iPSC-derived hepatocytes, fibroblasts and endothelial cells. Data are normalized to the housekeeper TBP.

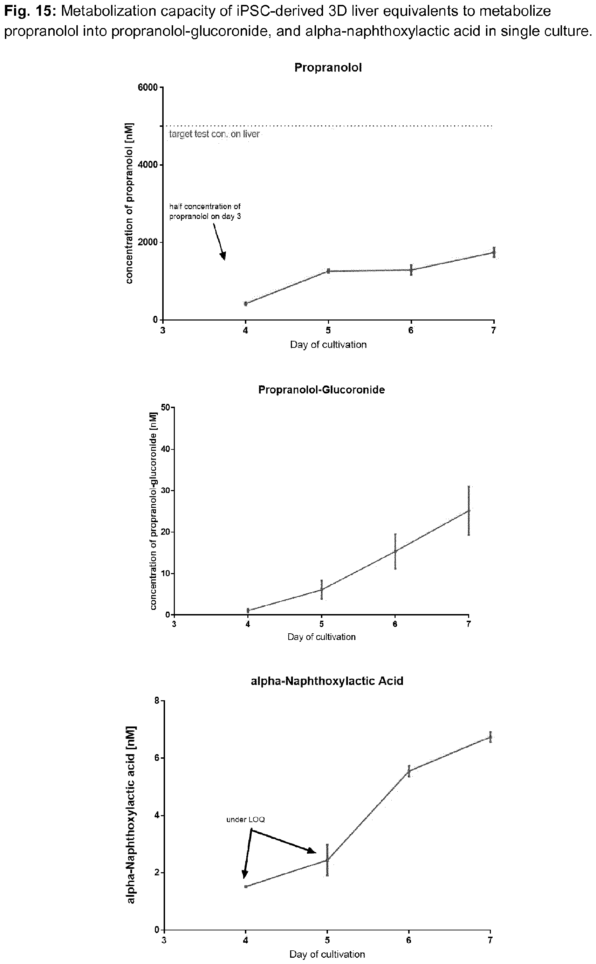

- Figure 15:: shows the metabolization capacity of iPSC-derived 3D liver equivalents to metabolize propranolol into propranolol-glucoronide, and alpha-naphthoxylactic acid, in single culture on a chip.

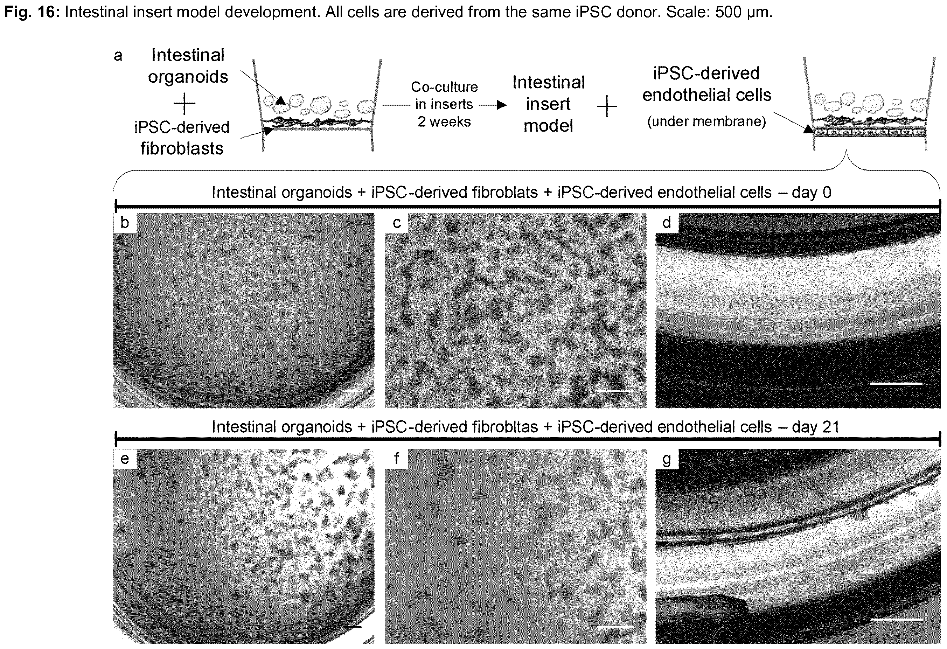

- Figure 16:: shows an intestinal insert model development. All cells are derived from the same iPSC donor. **a)**: Schematic of intestinal model set up; **b)** and **c)**: co-culture of iPSC-derived intestinal organoids and iPSC-derived fibroblasts after two weeks of static cultivation; **d)**: iPSC-derived endothelial cell layer visible underneath the insert membrane, under insert plastic edge; **e)** and **f)**: Additional three-week static cultivation of intestinal model with iPSC-derived organoids, fibroblasts and endothelial cells; **g)**: iPSC-derived endothelial cell layer under plastic insert edge after three weeks of static cultivation. Scale: 500 µm.

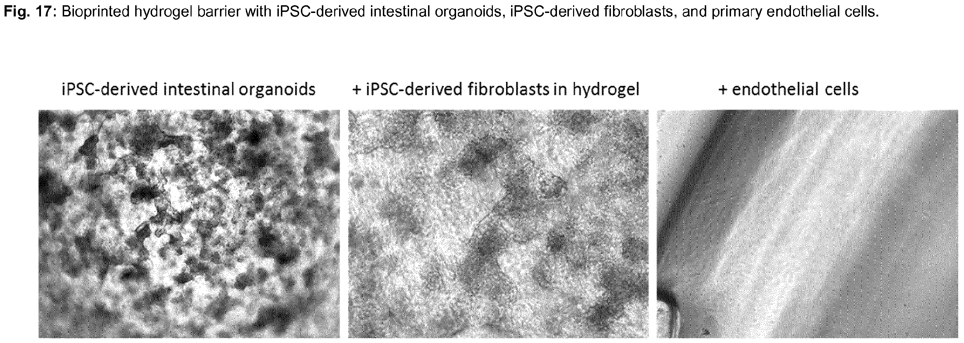

- Figure 17:: shows a bioprinted hydrogel barrier with iPSC-derived intestinal organoids, iPSC-derived fibroblasts, and primary endothelial cells.

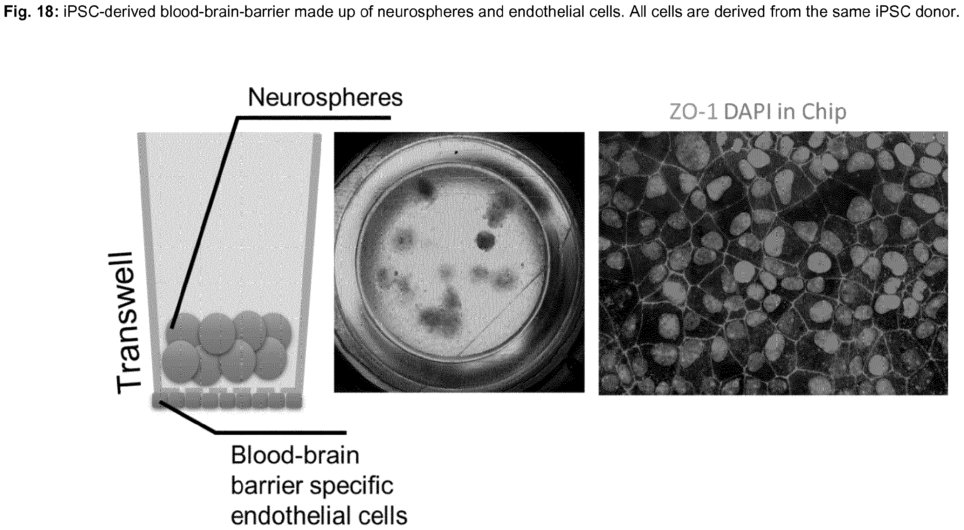

- Figure 18:: shows an iPSC-derived blood-brain-barrier made up of neurospheres and endothelial cells. All cells are derived from the same iPSC donor.

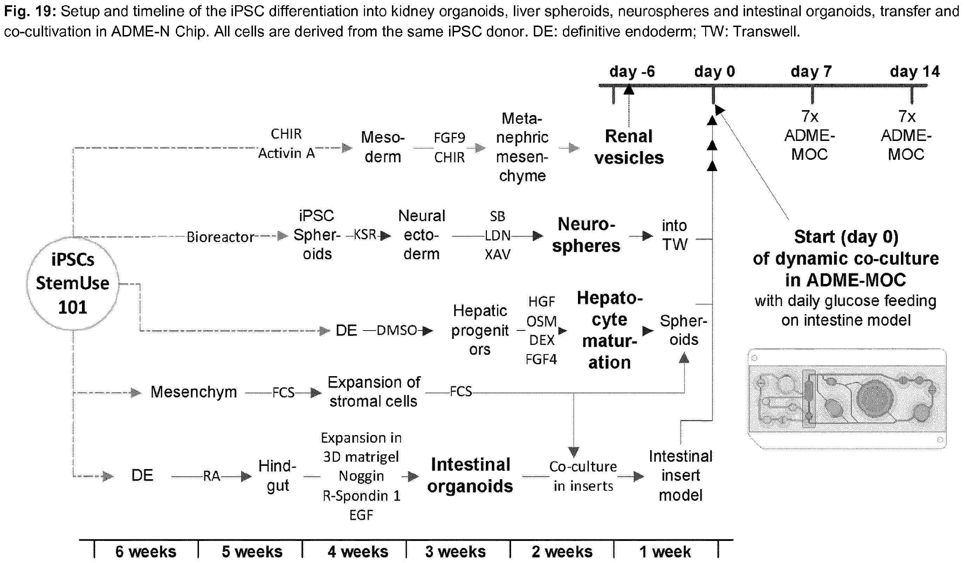

- Figure 19:: shows a setup and timeline of the iPSC differentiation into kidney organoids, liver spheroids, neurospheres and intestinal organoids, transfer and co-cultivation in ADME-N Chip. All cells are derived from the same iPSC donor. DE: definitive endoderm; TW: Transwell.

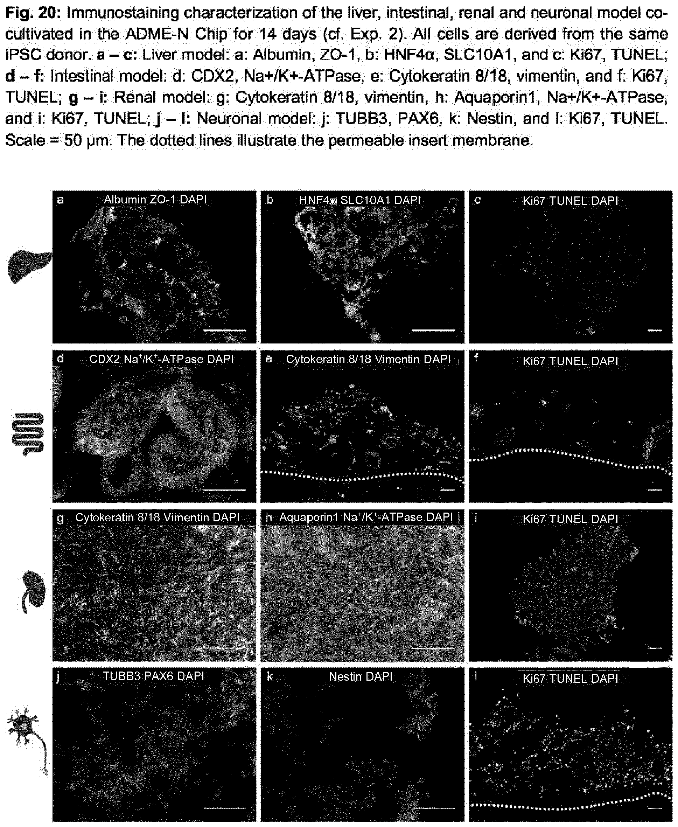

- Figure 20:: shows an immunostaining characterization of the liver, intestinal, renal and neuronal model co-cultivated in the ADME-N Chip for 14 days (cf. Experiment 2). All cells are derived from the same iPSC donor. **a) - c)**: Liver model: a: Albumin, ZO-1, b: HNF4α, SLC10A1, and c: Ki67, TUNEL; **d) - f)**: Intestinal model: d: CDX2, Na+/K+-ATPase, e: Cytokeratin 8/18, vimentin, and f: Ki67, TUNEL; **g) - i)**: Renal model: g: Cytokeratin 8/18, vimentin, h: Aquaporin1, Na+/K+-ATPase, and i: Ki67, TUNEL; j**) - l):** Neuronal model: j: TUBB3, PAX6, k: Nestin, and l: Ki67, TUNEL. Scale = 50 µm. The dotted lines illustrate the permeable insert membrane.
- Figure 21:: shows qPCR results of the co-culture in the ADME-N Chip of the iPSC-derived intestinal, liver, renal and brain model. All cells are derived from the same iPSC donor. Data are normalized to the housekeeper EF1 (a, c) or TBP (b, d). N = 3 - 6 for day 0, 7 and 14; N = 1 - 3 for iPSCs, and N = 1 for the positive controls represented. ND: no signal detected. Ordinary one-way ANOVA with Tukey's multiple comparisons test was used for statistical analysis. In each of a) to d): bars from left to right are "iPSCs", "Day 0", "Day 7", "Day 14", and the respective "positive control".

### DETAILED DESCRIPTON OF THE INVENTION

The present disclosure establishes the co-culturing of different induced pluripotent stem cell (iPSC)-derived organ precursor cells and/or iPSC-derived organ equivalent tissues in a microfluidic device. The novel microfluidic devices of the present disclosure realize multi-organ-chips with a number of organ equivalents that are specifically based on the differentiation of cells derived from a single donor iPSC that has in turn been reprogrammed from a single type of somatic cells. Thus, the iPSC-derived cells used in the present disclosure for differentiation into an organ equivalent include organ precursor cells, and more specifically precursor cells of organ-specific adult cells. Such iPSC-derived cells include, *e*.*g*., iPSC-derived hepatocyte precursor cells.

In various aspects, the present disclosure encompasses the use of iPSC-derived organoids, including, *e*.*g*., iPSC-derived hepatocyte organoids. In still other aspects, the present disclosure specifically encompasses the use of iPSC-derived neurospheres.

Experiment 2 of the present disclosure (*see* Figure 2 for the experimental setup of Experiments 1.1, 1.2 and 2) exemplifies the co-culturing of different iPSC-derived cells or organoids on a microfluidic chip. The cell types used in Example 2 reflect the organ types described for the claimed "iPSC multi-organ chip": hepatocytes (liver), intestinal cells (intestine), renal tubular cells (kidney), and neuronal cells (brain). Sets of chips were cultivated for 7, 14 and 21 days. At the endpoints, cellular samples were taken for immunohistological examinations, qPCR and RNA sequencing. Supernatants were analysed for their glucose, lactate, LDH (lactate dehydrogenase), albumin, urea, ALT (alanine aminotransferase) and AST (aspartate transaminase) content. Controls from static cultivations of the given organ equivalents were taken on day 0, 7 and 14.

The present disclosure relates to a microfluidic device comprising: (i) iPSC-derived hepatocyte precursor cells; (ii) iPSC-derived intestinal precursor cells; (iii) iPSC-derived renal tubular precursor cells; and (iv) iPSC-derived neuronal precursor cells; wherein the iPSC-derived precursor cells according to (i), (ii), (iii) and (iv) are obtained from a single donor of iPSC obtained from a single type of somatic cell.

As described herein, a precursor cell is derived from an iPSC, *i*.*e*., has been differentiated from an iPSC. The iPSC, in turn, is derived from a somatic cell, *i.e.*, has been reprogrammed from a somatic cell. For example, a hepatocyte precursor cell is derived from an iPSC, *i.e.*, has been differentiated from an iPSC, which in turn is derived from, *e*.*g*., a somatic skin cell, *i*.*e*., has been reprogrammed from a somatic skin cell.

As described herein, the cells may be derived (*i*.*e*., differentiated) from a single (*i*.*e*., one and the same) donor iPSC, which in turn has been obtained (*i*.*e*., reprogrammed) from a single type of somatic cell. For example, the cells may be derived (*i.e.*, differentiated) from a single (*i*.*e*., one and the same) iPSC, which has been obtained (*i*.*e*., reprogrammed) from, *e.g.*, a somatic skin cell, white blood cell, renal cell, adipocytes, or the like.

As described herein, a precursor cell, or more specifically an organ-specific precursor cell, which is derived from an iPSC, is by way of definition, no longer an iPSC, but may still show expression of one or more iPSC-specific markers, *e.g.*, expression of one or more reprogramming factors like OCT4, KLF4, SOX2, and optionally C-MYC. Specifically, a precursor cell as described herein is an organ precursor cell, which has been differentiated from an iPSC. Even more specifically, an organ-specific precursor cell as described herein is an organ-specific precursor cell, which has been differentiated from an iPSC. Thus, a precursor cell as described herein may be considered as a pre-differentiated organ cell, or a pre-differentiated immature organ cell. More specifically, an organ-specific precursor cell as described herein may be considered as a pre-differentiated an organ-specific cell, or a pre-differentiated immature an organ-specific cell.

In various embodiments, the precursor cell differentiated from an iPSC may be considered as an (iPSC-derived) induced organ-specific precursor cell.

Preferably, a precursor cell, or more specifically an organ-specific precursor cell, as described herein gives rise to a mature functioning organ cell. For example, a hepatocyte precursor cell as described herein (*i*.*e*., an iPSC-derived hepatocyte precursor cell) preferably gives rise to a mature functioning hepatocyte, but may also give rise to other hepatic cells. Thus, a precursor cell, or more specifically an organ-specific precursor cell, as described herein gives rise to a mature cell showing or retaining organ cell-specific functions. For example, a hepatocyte precursor cell as described herein preferably gives rise to a mature cell showing or retaining hepatocyte-specific functions. In various embodiments, a hepatocyte precursor cell as described herein gives rise to a mature cell showing or retaining specific functions of hepatic cells other than hepatocytes.

Accordingly, as described herein, mature hepatocytes obtained from iPSC-derived hepatocyte precursor cells are capable of, *inter alia*, recapitulating metabolic phenotypes observed in human populations, express drug-metabolizing enzymes at equivalent levels to primary human hepatocytes, and/or maintain stable functionality *in vitro.*

In various embodiments, the precursor cell differentiated from an iPSC may be considered as a mature organ cell-inducing cell in view of such precursor cells giving rise to a mature functioning organ cell. For example, an iPSC-derived hepatocyte precursor cell as described herein may be considered as a hepatocyte-inducing cell.

In various other embodiments, the precursor cell differentiated from an iPSC may be considered as an organoid-inducing cell in view of its capabilities to develop into adult-type organoids. For example, an iPSC-derived hepatocyte precursor cell as described herein may be considered as a hepatocyte organoid-inducing cell.

In various embodiments, precursor cells, or more specifically organ-specific precursor cells, as described herein may express one or more mature organ cell-specific markers or marker proteins. For example, in various embodiments of the present disclosure, hepatocyte precursor cells may express one or more mature hepatocyte-specific markers or marker proteins. In various embodiments, hepatocyte precursor cells may express cell markers including CD45 and CD109. In various embodiments, hepatocyte precursor cells may express cell markers including albumin, HNF4A, alpha-1-antitrypsin (AAT), and Alpha-fetoprotein (AFP), preferably albumin and/or AFP.

Likewise, in various embodiments, a mature organ cell as described herein express one or more mature organ cell-specific markers or marker proteins. For example, in various embodiments of the present disclosure, hepatocytes express one or more hepatocyte-specific marker proteins. In various embodiments, mature hepatocyte markers include albumin, PXR, H4 antigen, alpha-1-antitrypsin (AAT), cytokeratin 7 (CK7), cytokeratin 8 (CK8), hepatocyte nuclear transcription factor (HNF-3), CAAT enhancer-binding protein (CEBP)-alpha, CAAT enhancer-binding protein (CEBP)-beta, SLC10A1 (Sodium/bile acid cotransporter - liver bile acid transporter), HNF4a (Hepatocyte nuclear factor 4 alpha), Cytokeratin 8/18, CYP2A6, CYP2E1, and CYP3A4 (Cytochrome P450). Preferred mature hepatocyte markers include albumin, SLC10A1, HNF4a, Cytokeratin 8/18, CYP2A6, CYP2E1, and/or CYP3A4.

Drug metabolism is a central hepatocyte function. Terminal hepatocyte differentiation may be assessed by the expression and activity of drug-metabolizing enzymes in the cytochrome P450 (CYP) family. Thus, in various embodiments, mature hepatocyte markers include any one of CYP1A1, CYP1A2, CYP1B1, CYP2A6, CYP2A7, CYP2A13, CYP2B6, CYP2C8, CYP2C9, CYP2C18, CYP2C19, CYP2D6, CYP2E1, CYP2F1, CYP2J2, CYP2R1, CYP2S1, CYP2U1, CYP2W1, CYP3A4, CYP3A5, CYP3A7, CYP3A43, CYP4A11, CYP4A22, CYP4B1, CYP4F2, CYP4F3, CYP4F8, CYP4F11, CYP4F12, CYP4F22, CYP4V2, CYP4X1, CYP4Z2, CYP5A1, CYP7A1, CYP7B1, CYP8A1, CYP8B1, CYP11A1, CYP11B1, CYP11B2, CYP17A1, CYP19A1, CYP20A1, CYP21A1, CYP21A2, CYP24A1, CYP26A1, CYP26B1, CYP26C1, CYP27A1, CYP27B1, CYP27C1, CYP39A1, CYP46A1, and/or CYP51A1. In various embodiments, mature hepatocyte markers include any one of CYP1A2, CYP2C9, CYP3A4, Cytokeratin 8/18, CYP2A6, and/or CYP2E1. Preferred mature hepatocyte markers include Cytokeratin 8/18, CYP2A6, CYP2E1, and/or CYP3A4.

As further described herein, an iPSC-derived intestinal precursor cell preferably gives rise to a mature functioning intestinal cell. Thus, an iPSC-derived intestinal precursor cell preferably gives rise to a mature intestinal cell showing or retaining intestinal cell-specific functions. In various embodiments, an iPSC-derived intestinal precursor cell as described herein may express one or more (mature intestinal cell-specific and/or intestinal precursor cell-specific) marker proteins. In various embodiments, such mature intestinal cell-specific and/or intestinal precursor cell-specific marker proteins include ABCB1, CDH1, CHGA, DPP4, KL5, LGR5, Lyz, SLC2A1, SLC2A3, SOX9, and/or CXCR4.

In various embodiments, a mature intestinal cell as described herein is a mature cell of the small intestine. In various other embodiments, mature intestinal cells as described herein comprise mature epithelial cells of the intestine. In still other embodiments, mature intestinal cells as described herein comprise mature epithelial cells of the small intestine.

In various embodiments, a mature intestinal cell as described herein may express one or more mature intestinal cell-specific markers or marker proteins. In various embodiments, mature intestinal cell markers include CD326, CDX2, Cytokeratin 8/18, and/or NaK-ATPase (epithelial cells).

As further described herein, an iPSC-derived renal tubular precursor cell preferably gives rise to a mature functioning renal tubular cell, but may also give rise to other renal cells. Thus, an iPSC-derived renal tubular precursor cell preferably gives rise to a mature renal tubular cell showing or retaining renal tubular cell-specific functions. In various embodiments, a renal tubular precursor cell as described herein gives rise to a mature cell showing or retaining specific functions of renal cells other than renal tubular cells. In various embodiments, an iPSC-derived renal tubular precursor cell as described herein may express one or more (mature renal tubular cell-specific and/or renal tubular precursor cell-specific) marker proteins. In various embodiments, such mature renal tubular cell-specific and/or renal tubular precursor cell-specific marker proteins include Aquaporin1, MRP2, Cytokeratin 8/18, and/or NaK-ATPase.

In various embodiments, a mature renal tubular cell as described herein is a mature renal tubular epithelial cell. In various other embodiments, a mature renal tubular cell as described herein is a mature proximal renal tubular cell, preferably a mature proximal renal tubular epithelial cell. In further embodiments of the present disclosure, mature renal tubular cells as described herein comprise mature epithelial renal tubular cells, preferably mature epithelial proximal renal tubular cells.

In various embodiments, a mature renal tubular cell as described herein may express one or more mature renal tubular cell-specific markers or marker proteins. In various embodiments, mature renal tubular cell markers include Megalin (proximal tubular cells), Aquaporin1, MRP2, Cytokeratin 8/18, and/or NaK-ATPase.

As further described herein, an iPSC-derived neuronal precursor cell preferably gives rise to a mature functioning neuronal cell, but may also give rise to other cells of the brain. Thus, an iPSC-derived neuronal precursor cell preferably gives rise to a mature neuronal cell showing or retaining neuronal cell-specific functions. In various embodiments, an iPSC-derived neuronal precursor cell as described herein may express one or more (mature neuronal cell-specific and/or neuronal precursor cell-specific) marker proteins. In various embodiments, such mature neuronal cell-specific and/or neuronal precursor cell-specific marker proteins include Nestin (neuroectodermal stem cell marker), TUBB3 (Tubulin beta-3 chain, early biomarker of neural cell differentiation, and/or PAX6 (Paired box protein Pax-6, transcription factor, key regulatory gene of eye and brain development). In various other embodiments, such mature neuronal cell-specific and/or neuronal precursor cell-specific marker proteins may include GFAP, Synaptophysin, NeuN, and/or MAP2.

In various embodiments, a mature neuronal cell as described herein may express one or more mature neuronal cell-specific markers or marker proteins. In various embodiments, mature neuronal cell markers include NSE (gamma-enolase or enolase-2; a cytosolic protein expressed by mature neurons and cells of neuronal origin). In various embodiments, mature neuronal cell markers further include Nestin, TUBB3, and/or PAX6. In various other embodiments, mature neuronal cell markers include GFAP, Synaptophysin, NeuN, and/or MAP2.

The reprogramming of somatic cells has been established with the work of Yamanaka and Takahashi in 2006. As described herein, the somatic cell used for generating an iPSC can be any type of somatic cell reprogrammable to an iPSC, more any type of primary somatic cell. In various embodiments, fibroblasts are the primary somatic cell type used for the generation of iPSCs, preferably skin-derived fibroblasts. In various other embodiments, (hair-derived) keratinocytes are the primary somatic cell type used for the generation of iPSCs. In various embodiments, fibroblasts include skin cells, white blood cells, renal cells, adipocytes, and the like.

As described herein, iPSCs are characterized by the expression of reprogramming factors, including, *for example*, the main reprogramming factors OCT4, KLF4, SOX2, and optionally C-MYC. In various (preferred) embodiments, reprogramming factors identified for being expressed include SOX2, Nanog, OCT3/4, SSEA5, and/or TRA-1-60.

In the present disclosure, iPSCs are obtained from a single type of somatic cell by any reprogramming method available and known to the one of ordinary skill in the art. Menon et al. 2016 (Int. J. Mol. Sci., Vol. 17, 141) may provide an overview on iPSC reprogramming methods that may be used for reprogramming somatic cells into iPSCs. Kits and protocols for reprogramming somatic cells into iPSC are reported in the literature and commercially available.

As described herein, the iPSC obtained from a single type of somatic cell are available from a contract manufacturer. However, in various aspects, in particular aspects pertaining to the methods described herein, the present disclosure encompasses a step of reprogramming a (single type of) somatic cell into an iPSC.

In various embodiments, the term "iPSC obtained from (a single type of) somatic cell" means that the iPSC is reprogrammed from a somatic cell, which in turn may or may not have been obtained previously by differentiation of an iPSC.

In various embodiments, the iPSCs according to the present disclosure are obtained from adult somatic cells.

In various embodiments, the terms "somatic cells" and/or "adult somatic cells" may or may not encompass "somatic stem cells" and/or "adult somatic stem cells" and/or "adult stem cells". Preferably, the terms "somatic cells" or "adult somatic cells" exclude "somatic stem cells" and/or "adult somatic stem cells" and/or "adult stem cells".

In the present disclosure, iPSC-derived organ precursor cells can be obtained from the iPSCs by differentiation of iPSCs using means and methods for iPSC differentiation available and known to the one of ordinary skill in the art. Differentiation kits and protocols are reported in the literature and commercially available. Table 1 shows a list of iPSC-derived cells. Differentiation protocols were adapted from the listed papers.

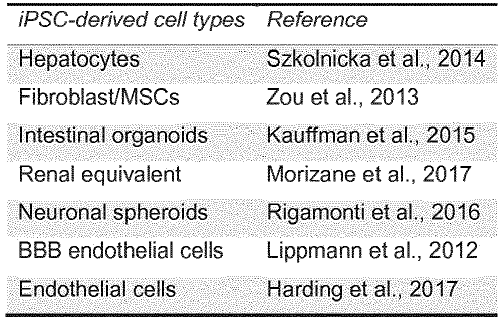

The differentiation protocols described in the scientific papers listed in Table 1 may be applied in the methods of the present disclosure.

As described herein, the iPSC-derived organ precursor cells are available from a contract manufacturer. However, in various aspects, in particular aspects pertaining to the methods described herein, the present disclosure encompasses a step of differentiating iPSCs as described herein into organ(-specific) precursor cells. As described elsewhere herein, such organ(-specific) precursor cells differentiated from iPSCs may be considered as pre-differentiated organ(-specific) cells, or pre-differentiated immature organ(-specific) cells.

As described herein, the microfluidic device may comprises: (i) iPSC-derived hepatocytes; (ii) iPSC-derived intestinal cells; (iii) iPSC-derived renal tubular cells; and (iv) iPSC-derived neuronal cells; wherein the iPSC-derived cells according to (i), (ii), (iii) and (iv) are obtained from a single donor of iPSC obtained from a single type of somatic cell.

As described herein, the microfluidic device may comprise: (i) iPSC-derived hepatocyte organoids; (ii) iPSC-derived intestinal organoids; and (iii) iPSC-derived renal tubular organoids; wherein the iPSC-derived organoids according to (i), (ii), and (iii) are obtained from a single donor of iPSC obtained from a single type of somatic cell.

The microfluidic device may further comprise (iv) iPSC-derived organoids of an additional organ or cell type, which are different from the organoids according to (i), (ii), and (iii), wherein the iPSC-derived organoids according to (i), (ii), (iii) and (iv) are obtained from a single donor of iPSC obtained from a single type of somatic cell.

The additional iPSC-derived organoids according to (iv) may be iPSC-derived neuronal organoids, wherein the iPSC-derived organoids according to (i), (ii), (iii) and (iv) are obtained from a single donor of iPSC obtained from a single type of somatic cell.

Similar to the disclosure pertaining to iPSC-derived organ precursor cells, an iPSC-derived organoid means an organoid obtainable or obtained by differentiation of iPSCs. As described elsewhere herein, an "organoid" may be considered to mean or represent the smallest functional organ or tissue unit. Accordingly, an organoid obtainable or obtained by differentiation of iPSCs specifically means differentiation of a single donor iPSC (obtained from a single type of somatic cell) and using the differentiated cell as a source for the organoid. More specifically, the said differentiated cells develop into organoids containing cells with organ-specific properties. The said differentiated cell may form spherical organoids that develop into adult-type organoids containing cells with organ-specific (functional) properties.

Preferably, the said organ-specific properties are organ-specific functional properties.

As further described herein, the microfluidic device may comprise: (i) iPSC-derived spherical organoids that develop into adult-type hepatocyte organoids; (ii) iPSC-derived spherical organoids that develop into adult-type intestinal organoids; and (iii) iPSC-derived spherical organoids that develop into adult-type renal tubular organoids, wherein the iPSC-derived spherical organoids according to (i), (ii), and (iii) are obtained from a single donor of iPSC obtained from a single type of somatic cell. The microfluidic device may further comprise (iv) iPSC-derived spherical organoids that develop into adult-type organoids that are different from the adult-type organoids according to (i), (ii), and (iii), wherein the iPSC-derived spherical organoids according to (i), (ii), (iii) and (iv) are obtained from a single donor of iPSC obtained from a single type of somatic cell.

The additional iPSC-derived spherical organoids according to (iv) may be iPSC-derived spherical organoids that develop into adult-type neuronal organoids, wherein the iPSC-derived spherical organoids according to (i), (ii), (iii) and (iv) are obtained from a single donor of iPSC obtained from a single type of somatic cell.

As described herein, like an organ-specific precursor cell disclosed herein, a cell differentiated from an iPSC and used as a source for an organoid as described above (differentiated cells develop into organoids containing cells with organ-specific properties), is by way of definition no longer an iPSC, but may still show expression of one or more iPSC-specific markers, *e*.*g*., expression of one or more reprogramming factors like OCT4, KLF4, SOX2, and optionally C-MYC. Reprogramming factors identified for being expressed include SOX2, Nanog, OCT3/4, SSEA5, and/or TRA-1-60. Specifically, such a differentiated cell may be considered as an (organ-specific) organoid precursor cell, which has been differentiated from an iPSC. Thus, a cell differentiated from an iPSC and used as a source for an organoid as described above, may be considered as a pre-differentiated cell, or a pre-differentiated immature cell, that develop into organoids containing cells with organ-specific properties, or that form spherical organoids that develop into adult-type organoids containing cells with organ-specific (functional) properties.

The cell differentiated from an iPSC and used as a source for an organoid as described above may be considered as an (iPSC-derived) induced organ-specific cell.

The cell differentiated from an iPSC and used as a source for an organoid as described above (differentiated cells develop into organoids containing cells with organ-specific properties) may be considered as an (iPSC-derived) organoid-inducing cell, or spherical organoid-inducing cell, in view of its capabilities to develop into adult-type organoids, or to form spherical organoids that develop into adult-type organoids.

As described herein, an iPSC-derived organoid according to the present disclosure may be capable of, *inter alia*, recapitulating metabolic phenotypes observed in human populations, express drug-metabolizing enzymes at equivalent levels to primary human hepatocytes, and/or maintain stable functionality *in vitro.*

In various embodiments, the organoid cells (*i.e.*, the cells making up an organoid) may express one or more mature organ cell-specific markers or marker proteins. For example, in various embodiments of the present disclosure, (iPSC-derived) hepatocyte organoid cells (*i*.*e*., cells making up a hepatocyte organoid) may express one or more mature hepatocyte-specific marker proteins. In various embodiments, hepatocyte organoid cells may express cell markers including CD45 and CD109. In various further embodiments, hepatocyte organoid cells may express cell markers including albumin, HNF4A, alpha-1-antitrypsin (AAT), and Alpha-fetoprotein (AFP), preferably albumin and/or AFP.

In still further embodiments, hepatocyte organoid cells may express cell markers including albumin, PXR, H4 antigen, AAT, CK7, CK8, HNF-3, CEBP-alpha, CEBP-beta, SLC10A1, HNF4a, Cytokeratin 8/18, CYP2A6, CYP2E1, and/or CYP3A4. Preferred markers include albumin, SLC10A1, HNF4a, Cytokeratin 8/18, CYP2A6, CYP2E1, and CYP3A4.

As further described herein, (iPSC-derived) intestinal organoid cells (*i.e.*, cells making up an intestinal organoid) as described herein may express one or more (mature intestinal cell-specific and/or intestinal precursor cell-specific) marker proteins. In various embodiments, such mature intestinal cell-specific and/or intestinal precursor cell-specific marker proteins include ABCB1, CDH1, CHGA, DPP4, KL5, LGR5, Lyz, SLC2A1, SLC2A3, SOX0, and/or CXCR4. In various other embodiments, an intestinal organoid cell as described herein may express mature intestinal cell markers including CD326, CDX2, Cytokeratin 8/18, and/or NaK-ATPase.

As further described herein, (iPSC-derived) renal tubular organoid cells (*i*.*e*., cells making up a renal tubular organoid) as described herein may express one or more (mature renal tubular cell-specific and/or renal tubular precursor cell-specific) marker proteins. In various embodiments, such mature renal tubular cell-specific and/or renal tubular precursor cell-specific marker proteins include Aquaporin1, MRP2, Cytokeratin 8/18, and/or NaK-ATPase.

As further described herein, (iPSC-derived) neuronal organoid cells (*i.e.*, cells making up a neuronal organoid) as described herein may express one or more (mature neuronal cell-specific and/or neuronal precursor cell-specific) markers or marker proteins. In various embodiments, such mature neuronal cell-specific and/or neuronal precursor cell-specific marker proteins include Nestin, TUBB3, and/or PAX6. In various other embodiments, such mature neuronal cell-specific and/or neuronal precursor cell-specific marker proteins may include GFAP, Synaptophysin, NeuN, and/or MAP2. In various embodiments, a neuronal organoid cell as described herein may express one or more mature neuronal cell-specific marker proteins. In various embodiments, mature neuronal cell markers include NSE. In various embodiments, mature neuronal cell markers further include Nestin, TUBB3, and/or PAX6. In various other embodiments, mature neuronal cell markers include GFAP, Synaptophysin, NeuN, and/or MAP2.

In the present disclosure, iPSC-derived cells used as a source for an organoid as described above (differentiated cells that develop into organoids containing cells with organ-specific properties) may be obtained from the iPSCs by differentiation of iPSCs using means and methods for iPSC differentiation available and known to the one of ordinary skill in the art. In various embodiments of the present disclosure, the iPSC-derived organoid-inducing cells (or iPSC-derived organoid precursor cells) are available from a contract manufacturer. However, in various aspects, in particular aspects pertaining to the methods described herein, the present disclosure encompasses a step of differentiating iPSCs as described herein into such organoid-inducing cells or iPSC-derived organoid precursor cells (*i*.*e*., cells used as a source for an organoid).

In various embodiments of the present disclosure, organoids are embedded into an extracellular matrix, preferably into an extracellular gel matrix, more preferably into a basement membrane-like extracellular matrix extract (typically resembling tissue extracellular environment). In various embodiments, the basement membrane-like extracellular (gel) matrix extract comprises a gelatinous protein mixture. Preferably, the gelatinous protein mixture is gelatinous protein mixture secreted by Engelbreth-Holm-Swarm (EHS) mouse sarcoma cells. More preferably, the basement membrane-like extracellular (gel) matrix is the Matrigel^{®} basement membrane matrix (Corning). In various embodiments of the present disclosure, the extracellular (gel) matrix is a hydrogel. In various embodiments of the present disclosure, the extracellular (gel) matrix is a collagen, agarose, aginate or Matrigel^{®} hydrogel. In various further embodiments, the hydrogel is a synthetic hydrogel.

In various embodiments of the present disclosure, the iPSC-derived precursor cells, or the iPSC-derived organoids, or the iPSC-derived cells used as a source for an organoid, are deposited in the microfluidic device in separate cell culture compartments.

In various embodiments, the microfluidic device may comprise a mixture of iPSC-derived cells and iPSC-derived organoids or spheroids. For example, the microfluidic device may comprise (i) iPSC-derived hepatocyte spheroids; (ii) iPSC-derived intestinal organoids; (iii) iPSC-derived renal tubular cells or iPSC-derived renal tubular precursor cells; and (iv) iPSC-derived neuronal spheroids; wherein the iPSC-derived spheroids, organoids and cells according to (i), (ii), (iii) and (iv) are obtained from a single donor of iPSC obtained from a single type of somatic cell. Preferably, the iPSC-derived spheroids, organoids and cells according to (i), (ii), (iii), and (iv) are deposited or present in separate cell culture compartments of the microfluidic chip. Preferably, the cell culture compartment comprising the iPSC-derived hepatocyte spheroids further comprises fibroblasts, preferably iPSC-derived fibroblasts. Accordingly, the iPSC-derived liver equivalent is accomplished by placing spheroids of iPSC-derived hepatocytes with (iPSC-derived) fibroblasts into the respective cell culture compartment.

In further preferred embodiments, the iPSC-derived intestinal organoids are embedded into an extracellular matrix or hydrogel as elsewhere herein. In other preferred embodiments, the cell culture compartment comprising the iPSC-derived intestinal organoids furthermore comprises fibroblasts and endothelial cells, preferably iPSC-derived fibroblasts and iPSC-derived endothelial cells, or iPSC-derived fibroblasts and primary microvascular endothelial cells. The primary microvascular endothelial cells or iPSC-derived endothelial cells are preferably seeded on the basolateral side of the cell culture compartment.

Furthermore, the cell culture compartment comprising iPSC-derived neuronal spheroids preferably further comprises a bioprinted blood brain barrier. More specifically, the cell culture compartment comprising iPSC-derived neuronal spheroids is enriched with endothelial cells, preferably iPSC-derived endothelial cells, seeded on the basolateral side of the cell culture compartment.

In various embodiments, the microfluidic device comprising cell culture compartments containing the iPSC-derived precursor cells, or the iPSC-derived organoids, or the iPSC-derived cells used as a source for an organoid, comprises a first circuit and a second circuit, wherein the cell culture compartments containing the iPSC-derived precursor cells, or the iPSC-derived organoids, or the iPSC-derived cells used as a source for an organoid, form part of the first circuit, and wherein the second circuit comprises a filtration unit and a reabsorption unit, wherein both circuits are connected with each other via the filtration unit and the reabsorption unit. Preferably, the filtration unit comprises a filtration barrier that selectively allows the passage of molecules from the first circuit to the second circuit based on size and charge of the molecules. More preferably, the filtration barrier is a biological barrier, even more preferably the biological barrier comprises podocytes. In further preferred embodiments, the reabsorption unit comprises a barrier that allows reabsorption of fluid from the second circuit to the first circuit. More preferably, the reabsorption barrier is a biological barrier, even more preferably the biological barrier comprises renal tubule cells.

In preferred embodiments, the microfluidic device comprises at least one cell culture compartment in the first circuit containing a liver equivalent or tissue mimicking liver function, and at least one cell culture compartment in the first circuit containing an intestine equivalent or tissue mimicking intestine function.

In various embodiments, the filtration unit and the reabsorption unit of the second circuit may be considered as kidney or renal equivalent. Preferably, the kidney or renal equivalent means a kidney or renal organoid. As described herein, a kidney or renal organoid preferably means organoids of podocytes and renal tubular cells, more preferably organoids of iPSC-derived podocytes and iPSC-derived renal tubular cells. Likewise, a liver organoid preferably means an organoid of hepatocytes, more preferably organoids of iPSC-derived hepatocytes. Likewise, a (small) intestine organoid preferably means an organoid of (small) intestinal cells, more preferably an organoid of iPSC-derived (small) intestinal cells. Likewise, a neuronal organoid preferably means an organoid of neuronal cells, more preferably an organoid of iPSC-derived neuronal cells. Likewise, respiratory tract (preferably lung) organoid preferably means an organoid of cells of the respiratory tract, preferably lung cells, more preferably lung epithelial cells. Preferably, the cells of the respiratory tract or the lung cells are iPSC-derived cells of the respiratory tract or iPSC-derived lung cells, respectively.

In various embodiments of the present disclosure, the novel microfluidic device has an arrangement of cell culture compartments such that the cell culture compartment comprising the "liver equivalent" is located downstream of the cell culture compartment comprising the "intestine equivalent" (in accordance with the layout for the "blood circuit" depicted in Fig. 1, however, without being limited thereto). Preferably, the cell culture compartments comprising the "liver equivalent" and the "intestine equivalent" are arranged in a separate microchannel, which is a branch of the main microchannel structure (in accordance with the layout for the "blood circuit" depicted in Fig. 1, however, without being limited thereto). Preferably, no other cell culture compartments are located between the cell culture compartments of the intestine equivalent and the liver equivalent. In various embodiments, the microfluidic device may comprise a branched microfluidic network (or branched network of microfluidic channels). In further preferred embodiments, the novel microfluidic device has an arrangement of cell culture compartments such that the cell culture compartment comprising the "neuronal equivalent" is arranged in parallel to the cell culture compartments comprising the "liver equivalent" and the "intestine equivalent", more preferably the "neuronal equivalent" is arranged in a separate microchannel branch arranged in parallel to the microchannel branch comprising the cell culture compartments comprising the "liver equivalent" and the "intestine equivalent" (in accordance with the layout for the "blood circuit" depicted in Fig. 1, however, without being limited thereto).

In case the microfluidic device contains a separate medium reservoir or sampling reservoir, this is preferably arranged in the main microchannel structure, *i*.*e*., is not arranged in a branch microchannel structure (in accordance with the layout for the "blood circuit" depicted in Fig. 1, however, without being limited thereto).

Furthermore, in case the microfluidic device contains a micropump, the micropump is preferably arranged in the main microchannel structure, *i*.*e*., is not arranged in a branch microchannel structure (in accordance with the layout for the "blood circuit" depicted in Fig. 1, however, without being limited thereto).

In various preferred embodiments, the second circuit does not show a branched microfluidic network, but rather is an unbranched circuit or circulation system. The terms "circuit" and "circulation system" may be used interchangeably herein.

In various embodiments of the present disclosure, a novel microfluidic device comprising: (i) iPSC-derived hepatocyte precursor cells; (ii) iPSC-derived intestinal precursor cells; (iii) iPSC-derived renal tubular precursor cells; and (iv) iPSC-derived neuronal precursor cells; wherein the iPSC-derived precursor cells according to (i), (ii), (iii) and (iv) are obtained from a single donor of iPSC obtained from a single type of somatic cell, has an arrangement of cell culture compartments in accordance with the layout shown in Fig. 1, however, without being limited thereto. The same holds for embodiments according to the present disclosure, wherein the microfluidic device comprises iPSC-derived organoids, or iPSC-derived cells used as a source for an organoid.

In various other embodiments of the present disclosure, a novel microfluidic device comprising: (i) iPSC-derived hepatocyte precursor cells; (ii) iPSC-derived intestinal precursor cells; (iii) iPSC-derived renal tubular precursor cells; and (iv) iPSC-derived neuronal precursor cells; wherein the iPSC-derived precursor cells according to (i), (ii), (iii) and (iv) are obtained from a single donor of iPSC obtained from a single type of somatic cell, has an arrangement of cell culture compartments in accordance with the layout for the "blood circuit" shown in Fig. 1, however, without being limited thereto. Such embodiments may or may not have one or more compartments, which match with the renal tubular and/renal glomerular equivalents of the urine circuit shown in Fig. 1 to provide an overlap of the two circuits. The same holds for embodiments according to the present disclosure, wherein the microfluidic device comprises iPSC-derived organoids, or iPSC-derived cells used as a source for an organoid.

As described herein, the novel microphysiological system of the novel microfluidic device comprising: (i) iPSC-derived hepatocyte precursor cells; (ii) iPSC-derived intestinal precursor cells; (iii) iPSC-derived renal tubular precursor cells; and (iv) iPSC-derived neuronal precursor cells; wherein the iPSC-derived precursor cells according to (i), (ii), (iii) and (iv) are obtained from a single donor of iPSC obtained from a single type of somatic cell, preferably is a closed channel system. The same holds for embodiments according to the present disclosure, wherein the microfluidic device comprises iPSC-derived organoids, or iPSC-derived cells used as a source for an organoid.

Accordingly, the blood and/or urine circuit of the novel microfluidic device form a closed circuit, more specifically a closed circuit of microfluidic channels. More specifically, the novel microphysiological system may be considered as a self-supported or self-contained microphysiological system. The closed channel system including the two renal functional units (filtration unit and reabsorption unit) enable specifically assaying kidney toxicity of test drugs. In preferred embodiments, the microphysiological system or microfluidic device is operated with a blood-mimicking liquid solution in the first circuit, and/or a urine mimicking liquid solution in the second circuit.

The novel microfluidic device comprising: (i) iPSC-derived hepatocyte precursor cells; (ii) iPSC-derived intestinal precursor cells; (iii) iPSC-derived renal tubular precursor cells; and (iv) iPSC-derived neuronal precursor cells; wherein the iPSC-derived precursor cells according to (i), (ii), (iii) and (iv) are obtained from a single donor of iPSC obtained from a single type of somatic cell, may comprise one or more cell culture compartments for each of the iPSC-derived precursor cells according to (i), (ii), (iii) and (iv).

Likewise, a novel microfluidic device comprising: (i) iPSC-derived hepatocyte organoids; (ii) iPSC-derived intestinal organoids; and (iii) iPSC-derived renal tubular organoids, wherein the iPSC-derived precursor cells according to (i), (ii), and (iii) are obtained from a single donor of iPSC obtained from a single type of somatic cell, may comprise one or more cell culture compartments for each of the iPSC-derived precursor cells according to (i), (ii), and (iii). In various embodiments, the microfluidic device further comprises (iv) iPSC-derived organoids of an additional organ or cell type, which are different from the organoids according to (i), (ii), and (iii), wherein the iPSC-derived organoids according to (i), (ii), (iii) and (iv) are obtained from a single donor of iPSC obtained from a single type of somatic cell. In preferred embodiments, the additional iPSC-derived organoids according to (iv) are iPSC-derived neuronal organoids, wherein the iPSC-derived organoids according to (i), (ii), (iii) and (iv) are obtained from a single donor of iPSC obtained from a single type of somatic cell.

In preferred embodiments, the novel microfluidic device comprises each organ equivalent only once, following the principle one individual organ equivalent per microfluidic device (or per microphysiological system). On the other hand, if, *for example*, at least one of the cell culture compartments of the first circuit of the novel microfluidic device comprises a liver equivalent, this means that there might be at least two cell culture compartments each comprising a liver equivalent.

In various embodiments, each cell culture compartment (or a single cell culture compartment) comprises only one organ equivalent, following the principle one individual organ equivalent per cell culture compartment.

Furthermore, in various embodiments, a cell culture compartment may comprise one or more organ equivalents, *e*.*g*., a neuronal equivalent along or in combination with an intestinal equivalent. In various other embodiments, each cell culture compartment (or a single cell culture compartment) comprises only one organ equivalent, *i*.*e*., an equivalent pertaining to one single organ, following the principle one individual organ equivalent per cell culture compartment.

The present disclosure further relates to a method of generating hepatocyte organoids, intestinal organoids, and renal tubular organoids, the method comprising co-culturing (i) iPSC-derived hepatocyte precursor cells, (ii) iPSC-derived intestinal precursor cells, and (iii) iPSC-derived renal precursor cells, in separate cell culture compartments that are in microfluidic connection with each other (via microfluidic channels).

The present disclosure also relates to a method of generating adult-type hepatocyte organoids, adult-type intestinal organoids, and adult-type renal tubular organoids, the method comprising co-culturing (i) iPSC-derived spherical organoids that develop into adult-type hepatocyte organoids, (ii) iPSC-derived spherical organoids that develop into adult-type intestinal organoids, and (iii) iPSC-derived spherical organoids that develop into adult-type renal organoids, in separate cell culture compartments that are in microfluidic connection with each other (via microfluidic channels).

In various aspects, the methods are carried out using a microfluidic device comprising the separate cell culture compartments that are in microfluidic connection with each other (via microfluidic channels).

In various aspects, the methods preferably further comprise co-culturing iPSC-derived endothelial cells, wherein the iPSC-derived endothelial cells are obtained from the same single donor of iPSC as the iPSC-derived precursor cells according to (i), (ii), and (iii), and wherein the iPSC-derived endothelial cells are deposited in the microfluidic channels connecting the cell culture compartments.

Preferably, the cell culture compartment containing the iPSC-derived hepatocyte precursor cells further comprises iPSC-derived fibroblasts, which are obtained from the same single donor of iPSC as the iPSC-derived hepatocyte precursor cells.

The novel microfluidic devices of the present disclosure can provide for organoid or tissue engineering with microphysiological *in vitro* systems using iPSC-derived organ-specific precursor cells, or iPSC-derived organ-specific organoids. The present disclosure further relates to novel bioengineered tissue constructs mimicking organ barriers generated by printing (bioprinting) iPSC-derived organoids in, and/or seeding on, a hydrogel. In particular, the present disclosure specifically relates to a bioengineered connective tissue construct mimicking mammalian organ barrier comprising iPSC-derived organoids, iPSC-derived fibroblasts, and iPSC-derived endothelial cells, and a bioengineered connective tissue construct mimicking the blood-brain-barrier comprising iPSC-derived neurospheres and iPSC-derived endothelial cells.

The iPSC chips of Experiment 1.1 and Experiment 2 of the present disclosure exemplify a bioengineered tissue constructs mimicking the intestinal barrier. In particular, the intestine equivalent of the iPSC chips of both said Experiments contains organoids of iPSC-derived intestinal cells embedded into Matrigel hydrogel containing also iPSC-derived fibroblasts and iPSC-derived endothelial cells. On the iPSC-derived intestinal equivalent model, primary microvascular endothelial cells or iPSC-derived endothelial cells are seeded on the basolateral side of the cell culture insert.

Furthermore, the iPSC chips of Experiment 1.2 contain a neuronal equivalent with blood brain barrier, with the cell culture insert containing iPSC-derived neuronal spheroids enriched with iPSC-derived endothelial cells printing (bioprinting) under the neuronal spheroids. In the neuronal equivalent with blood brain barrier of the iPSC chips of Experiment 2, the cell culture insert containing iPSC-derived neuronal spheroids is enriched with iPSC-derived endothelial cells seeded on the basolateral side of the cell culture insert.

Specifically, disclosed herein is a connective tissue construct mimicking mammalian organ-barrier, comprising: (i) iPSC-derived organoids; (ii) iPSC-derived fibroblasts; and (iii) iPSC-derived endothelial cells, wherein the iPSC-derived organoids and cells according to (i), (ii), and (iii) are obtained from a single donor of iPSC obtained from a single type of mammalian somatic cell. The connective tissue construct may be a bioengineered connective tissue construct. Furthermore, the mammalian organ barrier may be any of skin barrier, intestine barrier, respiratory tract barrier, e.g. lung barrier, eye barrier, oral mucosal tissue barrier, and genital mucosal tissue barrier, without being limited thereto. Accordingly, the iPSC-derived organoids may be any of iPSC-derived skin organoids, iPSC-derived intestine organoids, iPSC-derived organoids of the respiratory tract, preferably of the lung, iPSC-derived eye organoids, iPSC-derived oral mucosal tissue organoids, and iPSC-derived genital mucosal tissue organoids.

In various aspects, mimicking mammalian organ-barrier means mimicking mammalian organ-barrier function.

The connective tissue construct mimicking mammalian organ-barrier may be considered as a barrier organ equivalent.

In various aspects, the connective tissue construct is mimicking mammalian intestine barrier. Accordingly, described herein is a connective tissue construct mimicking mammalian intestine barrier, comprising: (i) iPSC-derived intestine organoids; (ii) iPSC-derived fibroblasts, preferably iPSC-derived intestinal fibroblasts; and (iii) iPSC-derived endothelial cells, preferably iPSC-derived intestinal endothelial cells, wherein the iPSC-derived intestine organoids and cells according to (i), (ii), and (iii) are obtained from a single donor of iPSC obtained from a single type of mammalian somatic cell.

Likewise, a connective tissue construct mimicking another mammalian organ barrier as described herein preferably comprises iPSC-derived organ-specific organoids; (ii) iPSC-derived fibroblasts, more preferably iPSC-derived organ-specific fibroblasts; and (iii) iPSC-derived endothelial cells, more preferably iPSC-derived organ-specific endothelial cells, wherein the iPSC-derived organ-specific organoids and (iPSC-derived) fibroblasts and endothelial cells according to (i), (ii), and (iii) are obtained from a single donor of iPSC obtained from a single type of mammalian somatic cell.

In various aspects, the (bioengineered) connective tissue construct mimicking mammalian organ-barrier comprises the (i) iPSC-derived organoids; (ii) iPSC-derived fibroblasts; and (iii) iPSC-derived endothelial cells printing (bioprinting) in, and/or seeding on, a hydrogel, wherein the iPSC-derived organoids and cells according to (i), (ii), and (iii) are obtained from a single donor of iPSC obtained from a single type of mammalian somatic cell.

The hydrogel may be an extracellular matrix, preferably an extracellular gel matrix, more preferably a basement membrane-like extracellular matrix extract (typically resembling tissue extracellular environment). The basement membrane-like extracellular (gel) matrix extract may comprise a gelatinous protein mixture. The gelatinous protein mixture may be gelatinous protein mixture secreted by Engelbreth-Holm-Swarm (EHS) mouse sarcoma cells. The basement membrane-like extracellular (gel) matrix may be the Matrigel^{®} basement membrane matrix (Corning). The extracellular (gel) matrix may be a collagen, agarose, aginate or Matrigel^{®} hydrogel. The hydrogel may be a synthetic hydrogel.

Further disclosed herein is a method of bioengineering or generating a connective tissue construct mimicking mammalian organ-barrier, the method comprising printing (bioprinting) in, and/or seeding on, a hydrogel: (i) iPSC-derived organoids, (ii) iPSC-derived fibroblasts, and (iii) iPSC-derived endothelial cells, wherein the iPSC-derived organoids and cells according to (i), (ii), and (iii) are obtained from a single donor of iPSC obtained from a single type of mammalian somatic cell. The hydrogel may be any hydrogel as described above.

The present disclosure relates to a connective tissue construct mimicking mammalian organ-barrier obtainable or obtained by the method of bioengineering or generating a connective tissue construct mimicking mammalian organ-barrier as described herein.

The present disclosure relates to the connective tissue construct mimicking mammalian organ-barrier described herein for use in any one of tissue repair, tissue engineering, and/or tissue manufacturing.

Disclosed herein is a tissue construct mimicking mammalian blood-brain-barrier comprising: (i) iPSC-derived neurospheres or iPSC-derived neuronal organoids; and (ii) iPSC-derived endothelial cells, wherein the iPSC-derived neurospheres or iPSC-derived neuronal organoids, and the iPSC-derived endothelial cells according to (i) and (ii) are obtained from a single donor of iPSC obtained from a single type of mammalian somatic cell. The tissue construct mimicking mammalian blood-brain-barrier may be considered as a bioengineered tissue construct mimicking mammalian blood-brain-barrier. The tissue construct mimicking mammalian blood-brain-barrier may be further comprise (iii) iPSC-derived fibroblasts; wherein the iPSC-derived neurospheres or iPSC-derived neuronal organoids, the iPSC-derived endothelial cells and the iPSC-derived fibroblasts according to (i), (ii) and (iii) are obtained from a single donor of iPSC obtained from a single type of mammalian somatic cell.

Also disclosed herein is a method of bioengineering or generating a tissue construct mimicking mammalian blood-brain-barrier, the method comprising printing (bioprinting) in, and/or seeding on, a hydrogel: (i) iPSC-derived neurospheres or iPSC-derived neuronal organoids; (ii) iPSC-derived endothelial cells; and (iii) iPSC-derived fibroblasts, wherein the iPSC-derived neurospheres or iPSC-derived neuronal organoids, the iPSC-derived endothelial cells, and the iPSC-derived fibroblasts according to (i), (ii), and (iii) are obtained from a single donor of iPSC obtained from a single type of mammalian somatic cell. The hydrogel may be any hydrogel as described above in connection with the tissue construct mimicking mammalian organ barrier.

In various aspects, mimicking mammalian blood-brain-barrier means mimicking mammalian blood-brain-barrier function.

The connective tissue construct mimicking mammalian blood-brain-barrier may be considered as a barrier organ equivalent.

Disclosed herein is a tissue construct mimicking mammalian blood-brain-barrier obtainable or obtained by the method of bioengineering or generating a tissue construct mimicking mammalian blood-brain-barrier as described herein.

Further disclosed herein is a microfluidic system comprising the connective tissue constructs mimicking mammalian organ barrier or mammalian blood-brain-barrier described herein.

Further disclosed herein is a method of bioengineering or generating an adult organ construct comprising co-culturing (i) iPSC-derived organ precursor cells, (ii) iPSC-derived fibroblasts, and (iii) iPSC-derived endothelial cells, in a single cell culture compartment, wherein the iPSC-derived organ precursor cells and iPSC-derived fibroblasts and endothelial cells according to (i), (ii), and (iii) are obtained from a single donor of iPSC obtained from a single type of mammalian somatic cell.

In various aspects, the method is a method of bioengineering or generating an adult liver organ construct comprising co-culturing (i) iPSC-derived hepatocyte precursor cells, (ii) iPSC-derived fibroblasts, and/or (iii) iPSC-derived endothelial cells, in a single cell culture compartment, wherein the iPSC-derived hepatocyte precursor cells and iPSC-derived fibroblasts and iPSC-derived endothelial cells according to (i), (ii), and/or (iii) are obtained from a single donor of iPSC obtained from a single type of mammalian somatic cell.

The iPSC-derived fibroblasts may be iPSC-derived hepatic fibroblasts. The iPSC-derived endothelial cells may be iPSC-derived hepatic endothelial cells.

The method of bioengineering or generating an adult organ construct may be carried out using a microfluidic device comprising a cell culture compartment for co-culturing (i) iPSC-derived organ precursor cells, (ii) iPSC-derived fibroblasts, and (iii) iPSC-derived endothelial cells.

The adult organ construct may be considered as an adult organ equivalent mimicking adult organ function.

As described herein, an adult organ construct may be characterized by expression of markers or marker proteins indicative of an adult organ, or an adult organ equivalent mimicking adult organ function. In various embodiments, an adult organ, or an adult organ equivalent mimicking adult organ function, can be considered as a mature organ, or a mature organ equivalent mimicking adult organ function. Marker proteins indicative of and expressed by mature organs, or mature organ equivalents mimicking adult organ function, are described elsewhere herein.

Disclosed herein is an adult organ construct obtainable or obtained by the method of bioengineering or generating an adult organ construct as described herein.

Specifically disclosed herein is an adult liver (organ) construct obtainable or obtained by the method of bioengineering or generating an adult liver (organ) construct as described herein.

Further disclosed herein is a novel method of bioengineering adult endothelial cells comprising exposing iPSC-derived endothelial precursor cells to shear stress. The method may comprise culturing the iPSC-derived endothelial precursor cells in the microfluidic channels of a microfluidic system. Fig. 11 of the present disclosure demonstrates that shear stress promotes elongation of iPSC-derived endothelial cells in the channels of the multi-organ-chip.

Disclosed herein is a method of bioengineering or generating adult endothelial cells comprising exposing iPSC-derived endothelial precursor cells to shear stress.

The method may comprise culturing the iPSC-derived endothelial precursor cells in the microfluidic channels of a microfluidic system.

Disclosed herein are adult endothelial cells obtainable or obtained by the method of bioengineering or generating adult endothelial cells as described herein.

The present disclosure further provides for implementing a blood circuit and a urine circuit in a microfluidic chip, and establishes a microphysiological system that supports physiological conditions to maintain a stable microenvironment in a system mimicking the interaction and crosstalk of a number of models emulating organ functionality. The novel microfluidic devices are suitable for assay establishment for pharmacokinetic-pharmacodynamic analysis in the context of ADME(T) profiling. Fig. 1 of the present disclosure shows an exemplary ADME-N (ADME + neuronal equivalent) chip footprint (top) and isometrical view on separate layers (bottom). The isometrical view exemplifies the positioning of a membrane between the two circuits (blood circuit and urine circuit). Fig. 12 shows a partial cross-sectional schematic view of an ADME chip of the present disclosure.

A novel microfluidic device implementing a blood circuit and a urine circuit as described herein has been shown to establish and maintain homeostatic stability of the microphysiological system, as evidenced by the glucose and LDH measurements depicted in Figures 3 and 10.

The glomerular and tubular compartments (filtration unit and reabsorption unit) can be accomplished with a combination of a polycarbonate membrane and a tight layer of renal cells (Fig. 4). The former implements a technical barrier to filter larger molecular weight medium constituents and retards their diffusion. The latter reabsorbs substances and causes the build-up of concentration gradients across the "blood" and "urine" circuit. This gradient is visible not only for LDH, but also for aspartate transaminase (AST, Fig. 5). Both of their distinctly different concentrations demonstrate the function of the renal membrane of the novel microfluidic ADME chip provided by the present disclosure. The AST originates from the liver equivalent as it is an intracellular enzyme found in hepatocytes.

All organ equivalents were immunohistochemically assessed to determine identity, functionality, and viability (Figs. 6-9).

Metabolic analysis revealed the establishment of a reproducible homeostasis between all organ equivalents. The present disclosure enables the demonstration of cell polarization, reorganization, barrier function, and trafficking that is observed *in vivo.* The achievement of a stable microenvironment in the novel microfluidic device combining a first circuit with one or more cell culture compartments and a second circuit comprising a filtration and reabsorption unit overlapping with the first circuit in the renal units opens possibilities for repeated dose testing, *e.g.*, in drug development.

Disclosed herein is a a novel microfluidic device comprising a first circuit with one or more cell culture compartments, and a second circuit comprising a filtration unit and a reabsorption unit, wherein both circuits are connected with each other via the filtration unit and the reabsorption unit. Figure 1 shows an exemplary layout of a novel microfluidic device comprising two circuits (termed "blood circuit" and "urine circuit") containing cavities for the incorporation of the following organ equivalents: intestine, liver, kidney (segregated into glomerulus and tubulus), and neuronal tissue. The former three tissues are used to accomplish the so-called ADME profile (adsorption, distribution, metabolism, excretion). The latter is an additional tissue supplementing that profile. The chip is, thus, termed ADME-N chip (ADME + neuronal equivalent/tissue). One reservoir compartment in each circuit allows sampling of supernatants ("medium reservoir 1 and 2"). The medium is perfused through the microfluidic network by two incorporated, pneumatic micropumps - one for each circuit. The circuits overlap in the two renal unit compartments and are separated by a porous membrane made of polycarbonate.

As compared to single-organ systems/chips, the microphysiological system/ microfluidic chip of the present disclosure can be considered as a multi-organ system/chip given that the microfluidic device comprises two circuits, and thereby comprises at least two cell culture compartments, with one of them being localized in the first circuit, and the other being one of the units of the second circuit mimicking renal function, in particular the reabsorption unit.

The novel microfluidic device may be considered as comprising a first circuit with one or more cell culture compartments, and a second circuit comprising a filtration unit and a reabsorption unit, wherein both circuits overlap in the filtration unit and the reabsorption unit. In various preferred embodiments, the overlapping parts of the two circuits (or the parts connecting both circuits with each other) are separated by a porous membrane. These overlapping/connecting parts specifically mean the filtration unit and the reabsorption unit.

The said porous membrane separating the overlapping/ connecting parts of the two circuits may be a synthetic or a biological porous membrane. The said porous membrane may be made of any of collagen, fibrin, or extracellular matrix (including decellularized organ matrix) without being limited thereto.

The porous membrane may exhibit the same properties as a Transwell^{®} insert. The porous membrane may be resorbable by cells.

The porous membrane may be made of polycarbonate.

The porous membrane may be a porous membrane made of poly(dimethylsiloxane) (PDMS).

The filtration unit and the reabsorption unit are separate cell culture compartments for incorporation of kidney cells, in particular a renal glomerular equivalent (filtration unit) and a renal tubular equivalent (reabsorption unit).

As described herein, the terms "cell culture compartment(s)" and "cell culture chamber(s)" or "cell culture cavity/ies" may be used interchangeably herein. A cell culture compartment according to the present disclosure is a compartment, chamber or cavity particularly suitable for culturing cells and tissues as used and described in the context of the present disclosure. In various embodiments, the cell culture compartment comprises a well plate format, in particular (micro)well cell culture plates (or multiwells) in any of 6-, 12-, 24-, 48-, 96-, and 384-well format. Preferred embodiments encompass multiwells in 24-, 48-, 96- or 384-well format. The multiwells can be made from a variety of materials, while the most common materials are polystyrene and polypropylene, which can be used in various embodiments of the present disclosure. In various other embodiments, the cell culture compartment is a Transwell^{®} (microfluidic) insert, more preferably a Transwell^{®} (microfluidic) insert made of polycarbonate (PC), polyester (PE), collagen-coated polytetrafluoroethylene (PTFE), or PET (polyethylene terephthalate). As described herein, a cell culture compartment used in the context of the present disclosure can be described to have a porous membrane (surface) for cell/tissue culture and growth.

As described herein, a porous membrane specifically means, without being limited thereto, a membrane having a porous structure that may be used not only for filtering, metering, and/or separating chemical and/or biological molecules, but also as a surface for cell or tissue culture and growth. The porous membrane of a cell culture compartment as used in the context of the present disclosure specifically serves to establish a fluid connection between the organ equivalent comprised by the cell culture compartment and the microfluidic network. Accordingly, in various embodiments, the porous membrane of a cell culture compartment as described herein is arranged such that the organ equivalent (cells/tissue) positioned on the surface of the porous membrane is in fluid connection with the microfluidic channels of the microfluidic device. In various embodiments, the porous membrane can be seeded with cells on both sides of the membrane. Preferably, endothelial cells are seeded at the bottom side of the porous membrane.

In general, through manufacturing processes the pore sizes can be tuned from a few nanometers to micrometers, thereby enabling the filtration, metering and separation of targeted chemical and biological molecules, and also the fluidic flow between the organ equivalents in the cell culture compartments and the microfluidic channel network. In various embodiments, the porous membrane is a porous membrane as described above. Thus, the porous membrane may be a synthetic or a biological porous membrane. In various embodiments, the said porous membrane may be made of any of collagen, fibrin, or extracellular matrix (including decellularized organ matrix) without being limited thereto. In various embodiments, the porous membrane exhibits the same properties as a Transwell^{®} insert. In various embodiments, the porous membrane is resorbable by cells. In various other embodiments, the porous membrane is made of polycarbonate. In various other preferred embodiments, the porous membrane is a porous membrane made of poly(dimethylsiloxane (PDMS).

As described herein, the term "first circuit" specifically means a circulatory system mimicking a mammalian blood circulatory system, more specifically mimicking the human blood circulatory system. As further described herein, the terms "first circuit" and "blood circuit equivalent" or "biomimetic blood circuit" may be used interchangeably herein. Likewise, as described herein, the term "second circuit" specifically means a circulatory system mimicking a mammalian urinary system, more specifically mimicking the human urinary system. As further described herein, the terms "second circuit" and "urine circuit equivalent" or "biomimetic urine circuit" may be used interchangeably herein.

Renal filtration includes both passive and active filtration, with the glomerulus acting as passive filtration barrier preventing larger molecular weight serum proteins from entering the nephron. Accordingly, such larger molecular weight serum proteins continue circulating in the blood stream. As described herein, the filtration unit of the novel microfluidic device disclosed herein comprises a filtration barrier that selectively allows the passage of molecules from the first circuit ("blood circuit") to the second circuit ("urine circuit") based on size and charge of the molecules, thereby mimicking the selective glomerulus filtration barrier.

In various embodiments of the present disclosure, the filtration barrier comprised by the filtration unit is established by the porous membrane separating the filtration unit (and the reabsorption unit) from the blood circuit (separating the overlapping parts of the two circuits). Thus, according to such embodiments, the filtration barrier may be considered a (porous) mechanical filtration barrier. Specifically, the (porous) mechanical filtration barrier is mimicking the glomerulus filtration barrier. Accordingly, in various embodiments, the filtration barrier may even be established by a cell culture compartment having a porous membrane. In preferred embodiments, the mechanical filtration barrier has a pore size of approximately 8 nm, but may have a pore size ≤ 8 nm. According to other preferred embodiments, the mechanical filtration barrier has a pore size setting an upper limit of about 69 kDa for serum proteins to enter the second (urine) circuit. In various embodiments, the mechanical filtration barrier has a pore size setting a limit for albumin, preferably serum albumin (or blood albumin), to enter the second (urine) circuit.

In various other embodiments of the present disclosure, the filtration barrier comprised by the filtration unit is a biological filtration barrier. Preferably, the biological filtration barrier is a barrier having filtration slits setting an upper limit of about 69 kDa for serum proteins to enter the second (urine) circuit. In various embodiments, the biological filtration barrier comprises podocytes (also termed visceral epithelial cells). More specifically, the biological filtration barrier is a podocyte (filtration) barrier preventing blood plasma proteins from entering the second (urine) circuit. The podocyte filtration barrier preferably has filtration slits setting an upper limit of about 69 kDa for serum proteins to enter the second (urine) circuit. In various embodiments, the biological or podocyte filtration barrier has a pore size setting a limit for albumin, preferably serum albumin (or blood albumin), to enter the second (urine) circuit.

In renal physiology, reabsorption (or tubular reabsorption) is the process by which the nephron removes water and solutes from the tubular fluid (pre-urine) and returns them to the circulating blood. The glomerular filtrate becomes more concentrated, which is one of the steps in forming urine. Reabsorption allows many useful solutes (primarily glucose and amino acids), salts and water to return to the blood circulation. As described herein, the reabsorption unit of the novel microfluidic device disclosed herein comprises a barrier that allows reabsorption of fluid from the second circuit (urine circuit) to the first circuit (blood circuit). In various embodiments of the present disclosure, the reabsorption barrier comprised by the reabsorption unit may be a (porous) mechanical barrier mimicking the reabsorption barrier. Preferably, the reabsorption barrier is a biological barrier, more preferably the biological reabsorption barrier comprises renal tubule cells (or renal tubular cells). More specifically, the biological reabsorption barrier is a renal tubular cell (reabsorption) barrier returning water, salt and solutes (the latter including proteins) into the first circuit (blood circuit). As described herein, reabsorption may be considered as the flow of glomerular filtrate from the reabsorption barrier (specifically from the tubular cells) into the first circuit (blood circuit), thereby allowing the selective passage of certain substances, in particular water, salt and solutes, back into the first circuit (blood circuit). The renal tubular cell may be considered as renal proximal tubular cell.

The second circuit of the novel microfluidic device disclosed herein may comprise a compartment, which does not serve as a cell culture compartment, but which serves as a (medium) reservoir compartment. Preferably, such a (medium) reservoir compartment may exclusively serve as a "sampling reservoir" or "sampling chamber/compartment" for taking samples from the second circuit, and/or which exclusively serves as a "sample reservoir" or "sample chamber/compartment" for adding a test sample to the second circuit of the microfluidic system. Accordingly, in preferred embodiments, the second circuit comprises no cell culture compartments other than the two compartments/units making up the filtration unit and the reabsorption unit, while including a separate (further) reservoir compartment, which serves to take samples (sampling compartment) from the second circuit, and/or which serves as a "sample reservoir" or "sample chamber/compartment" for adding a test sample to the second circuit of the microfluidic system.

The reservoir compartment may be used for adding a nutrient solution to the system, which is why it is also called a medium reservoir compartment. Accordingly, the reservoir compartment may serve three functions, sampling and feeding and adding test samples, however, does not serve as a cell culture compartment carrying an organ equivalent.

The first circuit of the novel microfluidic device disclosed herein may likewise comprise a compartment, which does not serve as a cell culture compartment, but which serves as a (medium) reservoir compartment. Preferably, such a (medium) reservoir compartment may exclusively serve to take samples from the first circuit. Also, such a reservoir compartment may be used for adding a nutrient solution to the system, which is why it may be called a medium reservoir compartment. Accordingly, the reservoir compartment of the first circuit may serve both functions, sampling and feeding, however, does not serve as a cell culture compartment carrying an organ equivalent. Accordingly, in preferred embodiments, the first circuit may comprise a reservoir compartment in addition to the one or more cell culture compartments, which serves to take samples (sampling compartment) from the first circuit and/or to add medium (nutrient solution) to the system.

As described herein, at least one of the cell culture compartments of the first circuit of the novel microfluidic device comprises a tissue mimicking liver function ("liver equivalent").

As further described herein, at least one of the cell culture compartments of the first circuit of the novel microfluidic device comprises a liver equivalent, and at least one of the cell culture compartments of the first circuit comprises an intestine equivalent. Such an embodiment is particularly useful to accomplish ADME profiling because the microfluidic chip then comprises organ equivalents of intestine and liver (first circuit), and kidney (second circuit). As described herein, the organ equivalents of intestine, liver, and kidney may be considered as "ADME" organ equivalents. In various embodiments, the novel microfluidic device may comprise one or more additional "non-ADME" organ equivalents, which are different from the "ADME" organ equivalents of intestine, liver, and kidney. In various embodiments, such additional "non-ADME" organ equivalents include, but are not limited thereto, a neuronal equivalent, a skin equivalent, or a respiratory tract equivalent (preferably lung equivalent).

In still other preferred embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a neuronal equivalent, thereby establishing an ADME-N chip.

In other preferred embodiments of the present disclosure, at least one of the cell culture compartments of the first circuit of the novel microfluidic device comprises a liver equivalent, and at least one of the cell culture compartments of the first circuit comprises a skin equivalent. Such an embodiment is also useful to accomplish ADME profiling because the microfluidic chip then comprises organ equivalents of skin and liver (first circuit), and kidney (second circuit). The administration of a test sample (test drug) is then performed via the skin equivalent. Such embodiments are particularly useful for testing cosmetics and/or consumer products, including creams.

In other preferred embodiments of the present disclosure, at least one of the cell culture compartments of the first circuit of the novel microfluidic device comprises a liver equivalent, and at least one of the cell culture compartments of the first circuit comprises a respiratory tract equivalent, specifically a lung equivalent. Such an embodiment is also useful to accomplish ADME profiling because the microfluidic chip then comprises organ equivalents of respiratory tract/lung and liver (first circuit), and kidney (second circuit). The administration of a test sample (test drug) is then performed via the respiratory tract/lung equivalent. Such embodiments are particularly useful for testing substances, including drugs, intended for application via the respiratory tract/lung.

In various other embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a skin equivalent.

In various further embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a respiratory tract, preferably lung, equivalent.

In still other embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a skin equivalent, and at least one of the cell culture compartments of the first circuit comprises a respiratory tract, preferably lung, equivalent.

In still further embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a skin equivalent, and at least one of the cell culture compartments of the first circuit comprises a respiratory tract, preferably lung, equivalent, and at least one of the cell culture compartments of the first circuit comprises a neuronal equivalent.

In various embodiments of the present disclosure, at least one of the cell culture compartments of the first circuit of the novel microfluidic device comprises a liver equivalent, and at least one of the cell culture compartments of the first circuit comprises an intestine equivalent, at least one of the cell culture compartments of the first circuit comprises a neuronal equivalent, and at least one of the cell culture compartments of the first circuit comprises a skin equivalent.

In various embodiments of the present disclosure, at least one of the cell culture compartments of the first circuit of the novel microfluidic device comprises a liver equivalent, and at least one of the cell culture compartments of the first circuit comprises an intestine equivalent, at least one of the cell culture compartments of the first circuit comprises a neuronal equivalent, and at least one of the cell culture compartments of the first circuit comprises a respiratory tract, preferably lung, equivalent.

Preferred organ equivalents include kidney equivalent, liver equivalent, intestine equivalent, preferably small intestine equivalent, neuronal (brain) equivalent, respiratory tract/lung equivalent, and skin equivalent. In case of kidney (or renal) equivalent, the equivalent specifically means an equivalent of the glomerulus and tubulus. More specifically, such equivalents of glomerulus and tubulus are arranged in separate units (filtration unit and reabsorption unit, respectively) of the second circuit of the novel microfluidic device provided herein.

As described herein, a "cell culture compartment" comprises or contains an organ equivalent, which encompasses cells and tissues mimicking organ function. Thus, in various embodiments, the terms "organ equivalent" and "cells/tissue mimicking organ function" may be used interchangeably herein.

In various embodiments, an organ equivalent or a tissue mimicking an organ function is a primary organ tissue, *e*.*g*., a primary liver tissue or a primary intestine tissue. Preferably, the primary organ tissue comprises epithelial cells of the respective organ, *i.e.*, epithelial cells of, *e.g.*, the gastrointestinal tract, the skin, or the respiratory tract.

In various embodiments, a tissue mimicking an organ function may be a tissue comprising epithelial cells of the respective organ, *i*.*e*., epithelial cells of, *e*.*g*., the gastrointestinal tract, the skin, or the respiratory tract. In various other embodiments, a tissue mimicking an organ function may be a tissue comprising cells of a cell line, including cells obtainable from a commercially available cell line.

In various other embodiments, a tissue mimicking organ function is a tissue comprising cells derived from induced pluripotent stem cells (iPSCs). Specifically, the cells may be derived (*i*.*e*., differentiated) from a single (*i*.*e*., one and the same) donor iPSC, which in turn has been obtained (*i*.*e*., reprogrammed) from a single type of somatic cell. For example, the cells may be derived (*i.e.*, differentiated) from a single (*i.e.*, one and the same) iPSC, which has been obtained (*i*.*e*., reprogrammed) from, *e*.*g*., a somatic skin cell, white blood cell, renal cell, adipocytes, or the like.

As described elsewhere herein, cells or tissue mimicking organ function include organoids and spheroids. Thus, in various embodiments, an organ equivalent specifically means an organoid or spheroid of a certain organ functionality, *e.g.*, an organoid of intestinal cells or hepatocytes, or a spheroid of intestinal cells or hepatocytes.

The cells, tissues and/or organs of the microfluidic device provided by the present disclosure can be modeled at a two-dimensional (2D), three-dimensional (3D), or organotypic complexity. 2D means suspension of monolayer cell cultures, while 3D includes, *e.g.*, multilayer cultures of different geometry, including, *e*.*g*., organoids and spheroids. Organotypic differs from 3D by capturing further features of the *in vivo* organ architecture.

In various embodiments, a cell culture compartment comprising an organ equivalent is a cell culture compartment comprising an organoid. As described herein, the term "organoid" may be considered to mean or represent the smallest functional organ or tissue unit. A selection of organoid histologies, all with relevant functionality and highly variable conglomerate geometry, has been published for 15 key human organs by Marx et al. (Marx et al. 2012, Altern Lab Anim 40, 235-257).

As further described herein, an organoid according to the present disclosure specifically means an organoid of iPSC-derived cells, however without being limited thereto. As further described herein, an organoid may be considered as a cell/tissue culture, which is mimicking organ function. As further described herein, an organoid may be typically characterized by any of the following characteristics, without being limited thereto: (i) (at least) three-dimensional multicellular construct or structure; (ii) collection of organ-specific cell types; (iii) if generated from stem cells (preferably iPSCs), it is usually recapitulating developmental organogenesis program; (iv) self-organization (ability of the cells to self-assemble or/and self-organize into tissues).

In various embodiments, a cell culture compartment comprising an organ equivalent is a cell culture compartment comprising spheroids mimicking organ function. As described herein, a spheroid according to the present disclosure specifically means a spheroid of iPSC-derived cells, however without being limited thereto. As further described herein, a spheroid may be considered as an aggregation or self-assembly of (spherical clusters of) cells, closely resembling *in vivo* organ tissues. A spheroid has, like an organoid, a three-dimensional structural architecture. As compared to organoids, it is considered that there is a higher order of self-assembly in organoids as opposed to spheroid cultures. In various embodiments, an organoid may be considered as a cell/tissue culture mimicking organ function.

Preferred organoids include kidney organoid, liver organoid, intestine organoid, preferably small intestine organoid, neuronal (brain) organoid, respiratory tract/lung organoid, and skin organoid. In case of a kidney (or renal) organoid, the organoid specifically means an organoid of the glomerulus and tubulus. More specifically, such organoids of glomerulus and tubulus are arranged in separate units (filtration unit and reabsorption unit, respectively) of the second circuit of the novel microfluidic device provided herein.

In various aspects of the present disclosure, at least one of the cell culture compartments of the first circuit of the novel microfluidic device comprises an iPSC-derived liver equivalent, and at least one of the cell culture compartments of the first circuit comprises an iPSC-derived intestine equivalent, and at least one of the cell culture compartments of the first circuit comprises an iPSC-derived neuronal equivalent, and the second circuit of the novel microfluidic device comprises renal proximal tubule epithelial cells (RPTEC), more specifically RPTECs seeded in the reabsorption unit. Preferably, the at least one cell culture compartment comprising an iPSC-derived intestine equivalent comprises organoids of iPSC-derived intestinal cells. More preferably, the organoids of iPSC-derived intestinal cells are embedded into an extracellular matrix, preferably into an extracellular gel matrix, more preferably into a basement membrane-like extracellular (gel) matrix extract (typically resembling tissue extracellular environment). In various embodiments, the basement membrane-like extracellular (gel) matrix extract comprises a gelatinous protein mixture. Preferably, the basement membrane-like extracellular matrix is the Matrigel^{®} basement membrane matrix (Corning).

In further preferred embodiments, the at least one cell culture compartment comprising an iPSC-derived intestine equivalent, specifically organoids of iPSC-derived intestinal cells, furthermore comprises iPSC-derived fibroblasts and iPSC-derived endothelial cells, or iPSC-derived fibroblasts and primary microvascular endothelial cells. The primary microvascular endothelial cells or iPSC-derived endothelial cells are preferably seeded on the basolateral side of the cell culture compartment.

Furthermore, the at least one cell culture compartment comprising an iPSC-derived neuronal equivalent preferably comprises iPSC-derived neuronal spheroids. More preferably, the at least one cell culture compartment comprising an iPSC-derived neuronal equivalent, specifically iPSC-derived neuronal spheroids, comprises a printed (bioprinted) blood brain barrier. More specifically, the at least one cell culture compartment comprising an iPSC-derived neuronal equivalent, specifically iPSC-derived neuronal spheroids, is enriched with endothelial cells, preferably iPSC-derived endothelial cells, printed (bioprinted) under the neuronal spheroids.

Furthermore, the at least one cell culture compartment comprising an iPSC-derived liver equivalent preferably comprises spheroids of iPSC-derived hepatocytes. More preferably, the at least one cell culture compartment comprising an iPSC-derived liver equivalent, specifically spheroids of iPSC-derived hepatocytes, further comprises fibroblasts, preferably iPSC-derived fibroblasts. In preferred embodiments, the iPSC-derived liver equivalent is accomplished by placing spheroids of iPSC-derived hepatocytes with iPSC-derived fibroblasts into the respective cell culture compartment.

In various embodiments, the RPTECs comprise a renal proximal tubule epithelial cell line.

In various aspects of the present disclosure, at least one of the cell culture compartments of the first circuit of the novel microfluidic device comprises primary liver tissue, and at least one of the cell culture compartments of the first circuit comprises a primary intestine tissue, preferably a primary small intestine tissue, and at least one of the cell culture compartments of the first circuit comprises an iPSC-derived neuronal equivalent, and the second circuit of the novel microfluidic device comprises renal proximal tubule epithelial cells (RPTECs), more specifically RPTECs seeded in the reabsorption unit and/or filtration unit. In various embodiments, the primary liver tissue comprises primary (human) hepatocytes. The primary liver tissue may be a commercially available liver model, *e.g.*, the 3D InSight^{™} Human Liver Microtissues XL, InSphero. In various embodiments, the primary liver tissue or liver model may comprise 50 spheroids, each containing 3,000 primary hepatocytes. Furthermore, in preferred embodiments, the primary intestine tissue comprises primary (human) cell-derived intestine epithelial and endothelial cells, and more preferably further comprises (human) fibroblasts. The primary intestine tissue may be a commercially available intestine model, *e*.*g*., the 3D Epilntestinal^{®} tissue model (MatTek Corporation). Furthermore, the at least one cell culture compartment comprising an iPSC-derived neuronal equivalent preferably comprises iPSC-derived neuronal spheroids. More preferably, the at least one cell culture compartment comprising an iPSC-derived neuronal equivalent, specifically iPSC-derived neuronal spheroids, comprises a printed (bioprinted) blood brain barrier. More specifically, the at least one cell culture compartment comprising an iPSC-derived neuronal equivalent, specifically iPSC-derived neuronal spheroids, is enriched with endothelial cells, preferably iPSC-derived endothelial cells, that are printed (bioprinted) under the neuronal spheroids.

In various embodiments, the RPTECs comprise a renal proximal tubule epithelial cell line.

In various aspects of the present disclosure, at least one of the cell culture compartments of the first circuit of the novel microfluidic device comprises an iPSC-derived liver equivalent, and at least one of the cell culture compartments of the first circuit comprises an iPSC-derived intestine equivalent, and at least one of the cell culture compartments of the first circuit comprises an iPSC-derived neuronal equivalent, and the second circuit of the novel microfluidic device comprises an iPSC-derived renal equivalent. Preferably, the at least one cell culture compartment comprising an iPSC-derived intestine equivalent comprises organoids of iPSC-derived intestinal cells. More preferably, the organoids of iPSC-derived intestinal cells are embedded into an extracellular matrix, preferably into an extracellular gel matrix, more preferably into a basement membrane-like extracellular (gel) matrix extract (typically resembling tissue extracellular environment). In various embodiments, the basement membrane-like extracellular (gel) matrix extract comprises a gelatinous protein mixture. Preferably, the basement membrane-like extracellular matrix is the Matrigel^{®} basement membrane matrix (Corning).

In further preferred embodiments, the at least one cell culture compartment comprising an iPSC-derived intestine equivalent, specifically organoids of iPSC-derived intestinal cells, furthermore comprises iPSC-derived fibroblasts and iPSC-derived endothelial cells, or iPSC-derived fibroblasts and primary microvascular endothelial cells. The primary microvascular endothelial cells or iPSC-derived endothelial cells are preferably seeded on the basolateral side of the cell culture compartment.

Furthermore, the at least one cell culture compartment comprising an iPSC-derived neuronal equivalent preferably comprises iPSC-derived neuronal spheroids. More preferably, the at least one cell culture compartment comprising an iPSC-derived neuronal equivalent, specifically iPSC-derived neuronal spheroids, comprises a printed (bioprinted) blood brain barrier. More specifically, the at least one cell culture compartment comprising an iPSC-derived neuronal equivalent, specifically iPSC-derived neuronal spheroids, is enriched with endothelial cells, preferably iPSC-derived endothelial cells, seeded on the basolateral side of the cell culture compartment.

Furthermore, the at least one cell culture compartment comprising an iPSC-derived liver equivalent preferably comprises spheroids of iPSC-derived hepatocytes. More preferably, the at least one cell culture compartment comprising an iPSC-derived liver equivalent, specifically spheroids of iPSC-derived hepatocytes, further comprises fibroblasts, preferably iPSC-derived fibroblasts. In preferred embodiments, the iPSC-derived liver equivalent is accomplished by placing spheroids of iPSC-derived hepatocytes with iPSC-derived fibroblasts into the respective cell culture compartment.

In various aspects of the present disclosure, at least one of the cell culture compartments of the first circuit of the novel microfluidic device comprises primary liver tissue, and at least one of the cell culture compartments of the first circuit comprises a primary intestine tissue, preferably a primary small intestine tissue, and at least one of the cell culture compartments of the first circuit comprises a primary neuronal tissue, and the second circuit of the novel microfluidic device comprises a primary renal tissue, preferably a primary renal tissue in the reabsorption unit, more preferably a primary tubular (epithelial cell) tissue in the reabsorption unit, and a primary glomerular tissue in the filtration unit. Preferably, the primary renal tissue comprises renal epithelial cells, more specifically renal proximal tubule epithelial cells (RPTECs), wherein the renal epithelial cells or RPTECs may be seeded in the reabsorption unit, and the primary glomerular tissue comprises podocytes, which may be seeded in the filtration unit. In various embodiments, the primary liver tissue comprises primary (human) hepatocytes. The primary liver tissue may be a commercially available liver model, *e*.*g*., the 3D InSight^{™} Human Liver Microtissues XL, InSphero. Furthermore, in preferred embodiments, the primary intestine tissue comprises primary (human) cell-derived intestine epithelial and endothelial cells, and more preferably further comprises (human) fibroblasts. The primary intestine tissue may be a commercially available intestine model, *e.g.*, the 3D Epilntestinal^{®} tissue model (MatTek Corporation). Furthermore, the primary neuronal tissue preferably comprises primary neuronal cells. In still further preferred embodiments, the least one cell culture compartment comprising the primary neuronal tissue, specifically primary neuronal cells, further comprises a printed (bioprinted) blood brain barrier. More specifically, the at least one cell culture compartment comprising the primary neuronal tissue, specifically primary neuronal cells, is enriched with endothelial cells, that may be either seeded on the basolateral side of the cell culture compartment, or are printed (bioprinted) under the neuronal spheroids.

In various aspects of the present disclosure, at least one of the cell culture compartments of the first circuit of the novel microfluidic device comprises a liver organoid, and at least one of the cell culture compartments of the first circuit comprises an intestine organoid, preferably a small intestine organoid, and the second circuit of the novel microfluidic device comprises a renal organoid in the reabsorption unit and/or the filtration unit. In various embodiments, at least one additional cell culture compartment of the first circuit further comprises an additional organoid, which is different from the liver organoid, the intestine organoid (including the small intestine organoid), and the renal organoid. Such an additional organoid may be considered as "non-ADME" organoid, while the liver organoid, the intestine organoid (including the small intestine organoid), and the renal organoid may be considered as "ADME" organoids. In various embodiments, such an additional (non-ADME) organoid may be any of a neuronal organoid, a skin organoid, or a respiratory tract (preferably lung) organoid. In various embodiments, at least two additional cell culture compartments of the first circuit further comprise two additional "non-ADME" organoids (one organoid per additional cell culture compartment), which are different from the liver organoid, the intestine organoid (including the small intestine organoid), and the renal organoid. In various embodiments, the two additional (non-ADME) organoids may be selected from any of a neuronal organoid, a skin organoid, and/or a respiratory tract (preferably lung) organoid. In still other embodiments, at least three additional cell culture compartments of the first circuit further comprise three additional "non-ADME" organoids (one organoid per additional cell culture compartment). In various embodiments, the three additional "non-ADME" organoids are a neuronal organoid, a skin organoid, and/or a respiratory tract (preferably lung) organoid. Preferably, the organoids are organoids of iPSC-derived organ cells. Specifically, the liver organoid preferably is an organoid of iPSC-derived hepatocytes, and the intestine organoid preferably is an organoid of iPSC-derived intestinal cells, more preferably an organoid of iPSC-derived cells of the small intestine, and the neuronal organoid preferably is an organoid of iPSC-derived neuronal cells, and the renal organoid preferably is an organoid of iPSC-derived renal cells.

In various aspects of the present disclosure, at least one of the cell culture compartments of the first circuit of the novel microfluidic device comprises a liver spheroid, and at least one of the cell culture compartments of the first circuit comprises an intestine spheroid, preferably a small intestine organoid, and the second circuit of the novel microfluidic device comprises a renal spheroid in the reabsorption unit and/or the filtration unit. In various embodiments, at least one additional culture compartment of the first circuit further comprises an additional spheroid, which is different from the liver spheroid, the intestine spheroid (including the small intestine spheroid), and the renal spheroid. Such an additional spheroid may be considered as "non-ADME" spheroid, while the liver spheroid, the intestine spheroid (including the small intestine spheroid), and the renal spheroid may be considered as "ADME" spheroids. In various embodiments, such an additional (non-ADME) spheroid may be any of a neuronal spheroid, a skin spheroid, and/or a respiratory tract (preferably lung) spheroid. In various embodiments, at least two additional cell culture compartments of the first circuit further comprise two additional "non-ADME" spheroids (one spheroid per additional cell culture compartment), which are different from the liver spheroid, the intestine spheroid (including the small intestine spheroid), and the renal spheroid. In various embodiments, the two additional (non-ADME) spheroids may be selected from any of a neuronal spheroid, a skin spheroid, and/or a respiratory tract (preferably lung) spheroid. In still other embodiments, at least three additional cell culture compartments of the first circuit further comprise three additional "non-ADME" spheroids (one spheroid per additional cell culture compartment). In various embodiments, the three additional "non-ADME" spheroids are a neuronal spheroid, a skin spheroid, and/or a respiratory tract (preferably lung) spheroid. Preferably, the spheroids are spheroids of iPSC-derived organ cells. Specifically, the liver spheroid preferably is a spheroid of iPSC-derived hepatocytes, and the intestine spheroid preferably is a spheroid of iPSC-derived intestinal cells, more preferably a spheroid of iPSC-derived cells of the small intestine, and the neuronal spheroid preferably is a spheroid of iPSC-derived neuronal cells, and the renal spheroid preferably is a spheroid of iPSC-derived renal cells.

In various embodiments, a cell culture compartment may comprise one or more organ equivalents, e.g., a neuronal equivalent along or in combination with an intestinal equivalent. In various other embodiments, each cell culture compartment (or a single cell culture compartment) may comprise only one organ equivalent, following the principle one individual organ equivalent per cell culture compartment. On the other hand, if, *for example*, at least one of the cell culture compartments of the first circuit of the novel microfluidic device comprises a liver equivalent, this means that there might be two cell culture compartments of the first circuit each comprising a liver equivalent. However, in preferred embodiments, the novel microfluidic device comprises each organ equivalent only once, following the principle one individual organ equivalent per microfluidic device (or per microphysiological system).

The present disclosure provides the use of a novel microfluidic device described herein in analytical testing, diagnostics, research, target validation, toxicity studies, tissue engineering, tissue manufacturing, drug screening, and/or pharmacokinetic-pharmacodynamic analysis, specifically ADME(T) profiling.

The present disclosure enables operating a microphysiological system and establishes the so-called ADME profiling based on a novel microfluidic device provided herein. Accordingly, the present disclosure provides a method of operating a microphysiological system making use of the novel microfluidic devices described herein, and further provides an ADME (absorption, distribution, metabolism, excretion) assay comprising the novel microfluidic devices. Further disclosed herein is an ADMET (absorption, distribution, metabolism, excretion, toxicity) assay comprising the novel microfluidic devices described herein.

Specifically in the drug discovery process, *in vitro* ADME profiling (or screening) provides a basis for selecting new molecular entities and lead compounds that have desirable drug metabolism, pharmacokinetics, or safety profiles, which are necessary for drug candidate selection and late-stage pre-clinical and clinical development. The ADME(T) profile (or properties) of a drug (test substance) allow the drug developer to understand the safety and efficacy data required for regulatory approval.

In the method of operating a microphysiological system, the culture time may last weeks or even months, enabling disease modeling and repeated dose substance exposure.

The present disclosure provides a method of performing an ADME (absorption, distribution, metabolism, excretion) profiling making use of the novel microfluidic device provided by the present disclosure. The present disclosure also provides a method of performing an ADMET (absorption, distribution, metabolism, excretion, toxicity) profiling making use of the novel microfluidic device described herein. Further disclosed herein is a method of generating an ADME profile, or an ADMET profile of a (test) drug comprising performing an ADME screening or ADMET screening, respectively, using the novel microfluidic device provided by the present disclosure.

The method of operating a microphysiological system as disclosed herein preferably comprises selectively adding a nutrient solution to the microphysiological system via the cell culture compartment comprising the gastrointestinal tract equivalent, preferably the intestine equivalent, more preferably the small intestine equivalent. The organ equivalent preferably comprises tissue comprising epithelial cells of the gastrointestinal tract, preferably of the intestine, even more preferably of the small intestine. In order to mirror physiological *in vivo* conditions, the present disclosure is preferably operating the microphysiological system with selectively adding the nutrient solution to the microphysiological system via the cell culture compartment comprising the gastrointestinal tract equivalent, preferably the intestine equivalent, more preferably the small intestine equivalent.

In various embodiments, a nutrient solution is selectively added to the microphysiological system via the cell culture compartment comprising the skin equivalent, and/or respiratory tract equivalent, more specifically lung equivalent.

As described herein, a nutrient solution specifically comprises substances (nutrients) which are essential for cells or a body to grow or to recover (including amino acids, salts (electrolytes), and glucose), and calories in the form of carbohydrates. Specifically, a nutrient solution as described herein is a nutrient solution for cell proliferation. As described herein, the terms "nutrient solution" and "cell culture medium" may be used interchangeably herein.

It has surprisingly been found that the novel microfluidic chips provided herein can establish homeostasis in the microphysiological system. This was shown by constant levels of glucose and LDH (Fig. 3). A constant glucose concentration suggests constant consumption and an active regulation of the available glucose. The glucose levels have reached equal levels in all compartments. It has furthermore surprisingly been found that feeding selectively through the intestine equivalent (apically through the intestine equivalent) was sufficient to supply both, the "blood" circuit and the "urine" circuit. Also, LDH activity has been shown rather constant throughout the experiments. As compared to glucose, LDH levels are different in each compartment. The barrier function of the intestine equivalent and the renal equivalent is shown by different concentrations of LDH in all three "compartments" (i.e., blood circuit -> medium reservoir 1; urine circuit -> medium reservoir 2; and intestine compartment). The barriers, thus, differentiate between the crossing substances. The active or passive penetration of glucose, while retaining other substances, indicate practical interactions between subsets of the chip system.

Accordingly, the present disclosure provides a method of mimicking homeostasis comprising operating a microphysiological system comprising a first circuit with one or more cell culture compartments and a second circuit comprising a filtration unit and a reabsorption unit, wherein both circuits are connected with each other via the filtration unit and the reabsorption unit. In accordance with the (preferred) embodiments described elsewhere herein with respect to the novel microfluidic device, the filtration unit comprised by the second circuit of the microphysiological system preferably comprises a filtration barrier that selectively allows the passage of molecules from the first circuit to the second circuit based on size and charge of the molecules. More preferably, the filtration barrier is a biological barrier, even more preferably wherein the biological barrier comprises podocytes.

Also in accordance with the (preferred) embodiments described elsewhere herein with respect to the novel microfluidic device, the reabsorption unit comprised by the second circuit of the microphysiological system preferably comprises a barrier that allows reabsorption of fluid from the second circuit to the first circuit. More preferably, the reabsorption barrier is a biological barrier, even more preferably wherein the biological barrier comprises renal tubule cells.

In various embodiments of the novel method of mimicking homeostasis, a nutrient solution is selectively added to the microphysiological system via the cell culture compartment (of the first circuit) comprising the gastrointestinal tract equivalent, more specifically the intestine equivalent, even more specifically the small intestine equivalent. In various embodiments, the organ equivalent comprises epithelial cells, or tissue comprising epithelial cells, of the gastrointestinal tract, preferably of the intestine, even more preferably of the small intestine.

The (preferred) embodiments described herein in relation to the novel microfluidic device likewise apply to the novel method of mimicking homeostasis comprising operating a microphysiological system as described herein. Thus, (preferred) embodiments described elsewhere herein in relation to the novel microfluidic device are also preferred embodiments in relation to the novel method of mimicking homeostasis comprising operating a microphysiological system. More specifically, the (preferred) embodiments described elsewhere herein in relation to the novel microfluidic device are also preferred embodiments of the microphysiological system comprised by the novel method of mimicking homeostasis.

The present disclosure also provides a method of detecting an analyte in a microphysiological system making use of a microfluidic device as described herein. In various embodiments, the method comprises adding a test sample to a cell culture compartment of the first circuit, preferably wherein the said cell culture compartment comprises an organ equivalent selected from any of gastrointestinal tract equivalent, more specifically intestine equivalent, skin equivalent, and/or respiratory tract equivalent, more specifically lung equivalent. The organ equivalent preferably comprises tissue comprising epithelial cells of the gastrointestinal tract, the skin, or the respiratory tract. In preferred embodiments, a test sample is added to a cell culture compartment comprising the gastrointestinal tract equivalent, more specifically the intestine equivalent. More preferably, the test sample is selectively added to cell culture compartment comprising the gastrointestinal tract equivalent, specifically the intestine equivalent, more specifically the small intestine equivalent.

In various embodiments, a test sample is selectively added to the microphysiological system via the cell culture compartment comprising the skin equivalent, and/or respiratory tract equivalent, more specifically the lung equivalent.

In various other embodiments, a test sample is selectively added to the microphysiological system via a cavity different from the cell culture compartments comprising the organ equivalents. Such a cavity may be the "medium reservoir" of the blood circuit and/or the urine circuit. As described elsewhere herein, such a "medium reservoir" is intended to be a "sampling reservoir" or "sampling chamber/compartment" which exclusively serves to take a sample or culture supernatant from the microfluidic system, and/or which is intended to be a "sample reservoir" or "sample chamber/compartment" which exclusively serves to add a test sample to the microfluidic system.

In various embodiments, the test sample is a liquid test sample, which is preferably added to the microphysiological system via the cell culture compartment comprising the gastrointestinal tract equivalent, specifically the intestine equivalent, more specifically the small intestine equivalent. In various embodiments, the test sample is a cosmetic or consumer product, including a cream, which is preferably added to the microphysiological system via the cell culture compartment comprising the skin equivalent.

The method of detecting an analyte according to the present disclosure may comprise taking a sample from a "medium reservoir" of the blood circuit and/or the urine circuit. As described elsewhere herein, such a "medium reservoir" may be intended to be a "sampling reservoir" or "sampling chamber/compartment" which serves to take a sample or culture supernatant from the microfluidic system, and/or which may be intended to be a "sample reservoir" or "sample chamber/compartment" which serves to add a test sample to the microfluidic system, but which does not serve as a cell culture compartment for an organ equivalent.

The method of detecting an analyte according to the present disclosure may further comprise analyzing the sample taken from the microfluidic system via a sampling chamber for the presence of metabolites, preferably toxic metabolites. The method of detecting an analyte according to the present disclosure specifically allows detecting metabolites of drugs added as test samples to the microphysiological system.

The method of detecting an analyte according to the present disclosure may further comprise detecting an analyte in the fluid flow of the first circuit and/or the second circuit.

In preferred embodiments of the method of detecting an analyte, a test sample is added to or deposited in a cell culture compartment of the first circuit, which is the same or different from the cell culture compartment to which a nutrient solution is added.

Further disclosed herein is a method of culturing and/or maintaining cells comprising seeding a novel microfluidic device described herein with cells.

Still further disclosed herein is a kit for operating a microphysiological system comprising a novel microfluidic device described, and instructions for use.

The microfluidic device provided by the present disclosure may be in form of a plate or a chip. Plates are usually based on the microtiter plate dimensions and use passive, gravity-based microfluidic flow or active on-board pumping. Chips of microscope slide size and other formats may be operated by an active or passive microfluidic flow, thereby emulating the shear stress at physiological intra-capillary or interstitial rates. In case of an active microfluidic flow, external or on-chip micropumps may be used. Preferably, the microfluidic device provided by the present disclosure is in form of a chip.

In various embodiments of the novel microfluidic device or the novel microphysiological system disclosed herein, the first circuit comprises one or more microfluidic channels that are lined with endothelial cells. As described elsewhere herein, the present disclosure demonstrates that shear stress promotes elongation of iPSC-derived endothelial cells in the channels of the multi-organ-chip (Fig. 11).

In various embodiments, the novel microfluidic device or the novel microphysiological system comprises a micropump, preferably a peristaltic micropump. In various embodiments, one of the two circuits (first and second circuit) may have a micropump (*i*.*e*., "blood circuit micropump" or "urine circuit micropump"). In preferred embodiments, each of the two circuits (first and second circuit) has its own micropump (*i*.*e*., "blood circuit micropump" and "urine circuit micropump"). The micropumps are preferably "on-chip" micropumps, in accordance with the preferred embodiment that the novel microfluidic device or the novel microphysiological system is a closed channel system.

As described herein, the novel methods provided herein may encompass a step of depositing and/or culturing an organ equivalent, preferably a tissue comprising (epithelial) cells or iPSC-derived (epithelial) cells of the respective organ, in a cell culture compartment of the first circuit.

In various embodiments, the intestine equivalent may (further) comprise fibroblasts, in particular iPSC-derived fibroblasts. Preferably, the intestine equivalent is an iPSC-derived intestine equivalent further comprising fibroblasts, in particular iPSC-derived fibroblasts. More preferably, the intestine equivalent is an iPSC-derived equivalent formed by a layer of an extracellular matrix, which is covered by fibroblasts, in particular iPSC-derived fibroblasts. The layer of an extracellular matrix preferably is a gel-based layer of an extracellular matrix. Even more preferably, the intestine equivalent is an organoid of iPSC-derived intestinal cells. Preferably, the intestine equivalent is an iPSC-derived equivalent, preferably an organoid of iPSC-derived intestinal cells, formed by a layer of a basement membrane-like extracellular matrix extract (typically resembling tissue extracellular environment), wherein the layer is covered by fibroblasts, in particular iPSC-derived fibroblasts. In various embodiments, the basement membrane-like extracellular matrix extract comprises a gelatinous protein mixture. Preferably, the basement membrane-like extracellular matrix is the Matrigel^{®} basement membrane matrix (Corning).

In various embodiments, the intestine equivalent, which preferably is an organoid of iPSC-derived intestinal cells, is incorporated or embedded into the aforementioned extracellular matrix.

In various embodiments, the intestine equivalent, which preferably is an organoid of iPSC-derived intestinal cells, comprises a (closed) layer of endothelial cells, including primary microvascular or iPSC-derived endothelial cells (the latter are preferred), on the basolateral side of the cell culture compartment. In various embodiments, the intestine equivalent, which preferably is an organoid of iPSC-derived intestinal cells, comprises a (closed) layer of iPSC-derived endothelial cells on the basolateral side of the cell culture compartment.

In various embodiments, the intestine equivalent may (further) comprise fibroblasts and endothelial cells, in particular iPSC-derived fibroblasts and iPSC-derived endothelial cells, as described above. Accordingly, in various embodiments, the intestine equivalent is an iPSC-derived intestine equivalent formed by a layer of an extracellular matrix, which is covered by fibroblasts, in particular iPSC-derived fibroblasts, and wherein the intestine equivalent further comprises another layer of endothelial cells, preferably primary microvascular endothelial cells, on the basolateral side of the cell culture compartment. Alternatively, in various embodiments, the intestine equivalent is an iPSC-derived intestine equivalent formed by a layer of an extracellular matrix, which is covered by fibroblasts, in particular iPSC-derived fibroblasts, and wherein the intestine equivalent further comprises another layer of iPSC-derived endothelial cells on the basolateral side of the cell culture compartment.

In various embodiments, the intestine equivalent is incorporated or embedded into the aforementioned extracellular matrix.

Furthermore, as described above, the iPSC-derived intestine equivalent preferably is an organoid of iPSC-derived intestinal cells. Also, the layer of the extracellular matrix preferably is a gel-based layer of an extracellular matrix, more preferably a layer of a basement membrane-like extracellular matrix extract (typically resembling tissue extracellular environment). The basement membrane-like extracellular matrix extract may comprise a gelatinous protein mixture. Preferably, the basement membrane-like extracellular matrix is the Matrigel^{®} basement membrane matrix (Corning).

In various embodiments, the neuronal equivalent may (further) comprise endothelial cells, in particular iPSC-derived endothelial cells. Preferably, the neuronal equivalent is an iPSC-derived equivalent further comprising endothelial cells, in particular iPSC-derived endothelial cells. More preferably, the endothelial cells, in particular iPSC-derived endothelial cells, are present on the basolateral side of the neuronal equivalent of the cell culture compartment comprising the neuronal equivalent. Alternatively, the neuronal equivalent is an iPSC-derived neuronal equivalent further comprising iPSC-derived endothelial cells printed (bioprinted) (and thus present) under the neuronal equivalent. Preferably, the iPSC-derived neuronal equivalent comprises spheroids or organoids of iPSC-derived neuronal cells, and the iPSC-derived endothelial cells are printed (bioprinted) (and thus present) under the neuronal spheroid or organoid of iPSC-derived neuronal cells. Preferably, the iPSC-derived neuronal equivalent comprises spheroids or organoids of iPSC-derived neuronal cells, which are incorporated or embedded into a layer of an extracellular matrix. Preferably, the iPSC-derived endothelial cells are printed (bioprinted) (and thus present) in the extracellular matrix under (basolateral) the neuronal spheroids or organoids of iPSC-derived neuronal cells. As described above, the extracellular matrix preferably is an extracellular gel matrix, more preferably a basement membrane-like extracellular matrix extract (typically resembling tissue extracellular environment). The basement membrane-like extracellular matrix extract may comprise a gelatinous protein mixture. Preferably, the basement membrane-like extracellular matrix is the Matrigel^{®} basement membrane matrix (Corning).

The endothelial cells printed (bioprinted) under the neuronal spheroids or organoids, or seeded on the basolateral side of the neuronal equivalent, in particular on the basolateral side of the cell culture compartment comprising the neuronal equivalent, resemble the blood-brain-barrier, and its tightness has been shown by a TEER of 20-78 Ω x cm² after two weeks of chip cultivation. Transepithelial/transendothelial electrical resistance (TEER) is a widely accepted quantitative technique to measure the integrity of tight junction dynamics in cell culture models of endothelial and epithelial cell layers. TEER is a strong indicator of the integrity of the cellular barriers before they are evaluated, e.g., for transport of drugs or chemicals. TEER measurements can be performed in real time without cell damage and generally are based on measuring ohmic resistance or measuring impedance across a wide spectrum of frequencies. The measurements for various cell types have been reported with commercially available measurement systems and also with custom-built microfluidic implementations. Some of the barrier models that have been widely characterized using TEER include the blood-brain barrier (BBB), gastrointestinal (GI) tract, and pulmonary models.

In various embodiments of the present disclosure, endothelial cells printed (bioprinted) under the neuronal spheroids or organoids, or seeded on the basolateral side of the neuronal equivalent, in particular on the basolateral side of the cell culture compartment comprising the neuronal equivalent, show a TEER of 20-78 Ω x cm², specifically after two weeks of chip cultivation.

In various embodiments of the present disclosure, the cells of the intestinal equivalent are characterized by expression of specific markers of intestinal epithelial cells, including including sucrase-isomaltase, the intestinal oligopeptide transporter SLC15A1/peptide transporter 1 (PEPT1), and the major metabolizing enzyme CYP3A4.

In various embodiments of the present disclosure, the cells of the intestinal equivalent and/or the liver equivalent are characterized by expression of specific markers including the major metabolizing enzyme CYP3A4.

In various embodiments of the present disclosure, the cells of the liver equivalent are characterized by expression of specific markers including cytokeratin 8/18.

In various embodiments of the present disclosure, the liver equivalent is characterized by expression of tight junction ZO-1 protein.

In various embodiments of the present disclosure, the cells of the neuronal equivalent are characterized by expression of specific markers including beta-tubulin III.

In various embodiments of the present disclosure, (primary) endothelial cells are characterized by expression of CD31 and/or von-Willebrand-Faktor (vWF).

In various embodiments of the present disclosure, (iPSC-derived) fibroblasts are characterized by expression of vimentin.

As described herein, any type of cells used and described in the present disclosure may be derived from induced pluripotent stem cells (iPSCs). Specifically, the cells may be derived (*i*.*e*., differentiated) from a single (*i.e.*, one and the same) donor iPSC, which in turn has been obtained (*i*.*e*., reprogrammed) from a single type of somatic cell. For example, the cells may be derived (*i.e.*, differentiated) from a single (*i.e.*, one and the same) iPSC, which has been obtained (*i.e.*, reprogrammed) from, *e.g.*, a somatic skin cell, white blood cell, renal cell, adipocytes, or the like. Thus, to the extent that the cells are not already identified as iPSC-derived cells, cells used and described in the present disclosure may be iPSC-derived cells according to various embodiments, preferably iPSC-derived cells that have been differentiated from a single (*i.e.*, one and the same) donor iPSC, which has been obtained (*i.e.*, reprogrammed) from a single type of somatic cell.

As described herein, reference to the "intestine" means according to preferred embodiments reference to the "small intestine". For example, reference to "intestine equivalent" or "cells of the intestine" means according to preferred embodiments reference to "small intestine equivalent" or "cells of the small intestine", respectively.

As described herein, reference to "cells" encompasses reference to "mammalian cells", while reference to "human cells" is preferred. For example, reference to "endothelial cells" encompasses reference to "mammalian endothelial cells", while reference to "human endothelial cells" is preferred.

As further described herein, reference to "tissue" encompasses reference to "mammalian tissue", while reference to "human tissue" is preferred. For example, reference to "liver tissue" encompasses reference to "mammalian liver tissue", while reference to "human liver tissue" is preferred.

As further described herein, in various embodiments, the term "tissue" may be considered to be a "tissue culture".

Furthermore, as described herein, "culturing cells" preferably means "fluidic culturing of cells".

In various embodiments, the present disclosure encompasses fluidic (or fluid) shear stress, specifically physiologically relevant fluidic (or fluid) shear stress.

### EXAMPLES

### Chip design of ADME-N Chip

A chip design is realized having constraints scaled down from human physiology. Dimensional data as well as flow characteristics are considered. The layout comprises two circuits (termed "blood" and "urine") containing cavities for the incorporation of the following organ equivalents: intestine, liver, kidney (segregated into glomerulus and tubulus), and neuronal tissue (Fig. 1). The former three tissues are used to accomplish the so-called ADME profile (adsorption, distribution, metabolism, excretion). The latter is an additional tissue supplementing that profile. The chip is, thus, termed ADME-N chip. One reservoir compartment in each circuit allows sampling of supernatants (medium reservoir 1 and 2). The medium is perfused through the microfluidic network by two incorporated, pneumatic micropumps - one for each circuit. The circuits overlap in the two kidney compartments and are separated by a porous membrane made of polycarbonate.

### Intestine compartment

The intestine equivalent was realized by placing a 24-well standing cell culture insert into the respective cavity (cell culture compartment). The insert contained either a commercially available intestinal model (Epilntestinal^{®}, MatTek) or organoids of iPSC-derived intestinal cells embedded into Matrigel containing also iPSC-derived fibroblasts and iPSC-derived endothelial cells. On the iPSC derived intestinal equivalent model, primary microvascular endothelial cells or iPSC-derived endothelial cells were seeded on the basolateral side of the 24-well standing cell culture insert. The apical side of the insert was used for the daily feeding of a nutritious solution (Nutriflex^{®} peri, B.Braun). In total, the apical side contained a supernatant of 250 µL.

### Neuronal compartment

The neuronal equivalent with blood brain barrier consisted of a 96-well hanging cell culture insert containing iPSC-derived neuronal spheroids enriched with iPSC-derived endothelial cells seeded on the basolateral side of the 96-well hanging cell culture insert or bioprinted under the neuronal spheroids. The apical side of the insert contained a volume of 75 µL.

### Liver compartment

The liver equivalent was accomplished either by placing commercially available liver models (3D InSight^{™} Human Liver Microtissues XL, InSphero) or spheroids of iPSC-derived hepatocytes with iPSC-derived fibroblasts into the respective cavity. Of the former, 50 spheroids were placed into one chip, each containing 3,000 primary hepatocytes. Of the latter, 20 spheroids were placed into one chip, each containing 50,000 cells.

### Kidney (renal) compartment

The kidney compartments were seeded either with a renal proximal tubule epithelial cell line (RPTECs) or with iPSC-derived renal cells.

The used differentiation protocols for the iPSC-derived cells were adapted from different papers listed in Table 1.

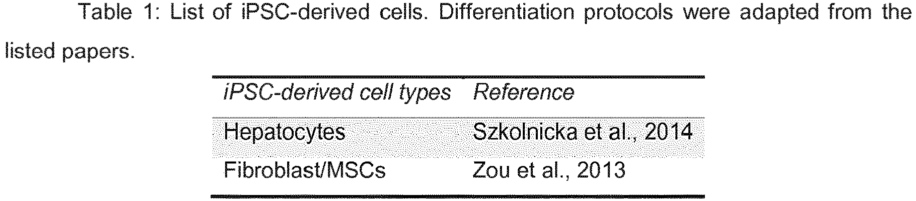

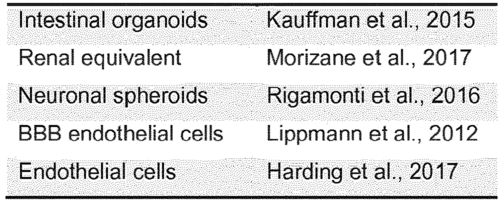

The "blood" circuit had an overall volume of approximately 1.37 mL (not including apical volumes). The "urine" circuit had an overall volume of approximately 0.58 mL. Initially, the chip contained medium with glucose and serum. Every day, samples were taken from both medium reservoirs and from the apical side of the intestine equivalent. The taken volumes were replaced with glucose-free medium for the "blood" medium reservoir and glucose- and serum-free medium for the "urine" medium reservoir. To the intestinal compartment a glucose-rich Nutriflex^{®} peri solution was given. In this way, 1.0 mg of glucose was fed daily to the system only through the intestine equivalent.

Sets of chips were cultivated for 7, 14 and 21 days (Fig. 2). At the endpoints, cellular samples were taken for immunohistological examinations, qPCR and RNA sequencing. Supernatants were analysed for their glucose, lactate, LDH, albumin, urea, ALT and AST content. Controls from static cultivations of the given organ equivalents were taken on day 0, 7 and 14.

### Experiment 1.1: iPSC Chips - 3 chips

- iPSC liver equivalent
- iPSC intestine equivalent
- iPSC neuronal equivalent with blood brain barrier printed
- RPTECs kidney

### Intestine compartment

The insert of the intestine equivalent contains organoids of iPSC-derived intestinal cells embedded into Matrigel containing also iPSC-derived fibroblasts and iPSC-derived endothelial cells. On the iPSC derived intestinal equivalent model, primary microvascular endothelial cells or iPSC-derived endothelial cells are seeded on the basolateral side of the 24-well standing cell culture insert.

### Neuronal compartment

The neuronal equivalent with blood brain barrier consists of a cell culture insert containing iPSC-derived neuronal spheroids enriched with iPSC-derived endothelial cells bioprinted under the neuronal spheroids.

### Liver compartment

The liver equivalent is accomplished by placing spheroids of iPSC-derived hepatocytes with iPSC-derived fibroblasts into the respective cavity. 20 spheroids are placed onto one chip, each containing 50,000 cells (24:1 ratio of hepatocytes:fibroblasts).

### Kidney (renal) compartment

The kidney compartments are seeded with a renal proximal tubule epithelial cell line (RPTECs).

### Experiment 1.2: Primary tissue Chips - 10 chips

- Primary liver tissue
- Primary small intestine
- iPSC neuronal equivalent with blood brain barrier printed
- RPTECs kidney

### Intestine compartment

The insert of the intestine equivalent contains a commercially available intestinal model (Epilntestinal^{®}, MatTek).

### Neuronal compartment

The neuronal equivalent with blood brain barrier consists of a cell culture insert containing iPSC-derived neuronal spheroids enriched with iPSC-derived endothelial cells bioprinted under the neuronal spheroids.

### Liver compartment

The liver equivalent is accomplished by placing commercially available liver models (3D InSight^{™} Human Liver Microtissues XL, InSphero) into the respective cavity. 50 spheroids are placed onto one chip, each containing 3,000 primary hepatcytes.

### Kidney (renal) compartment

The kidney compartments are seeded with a renal proximal tubule epithelial cell line (RPTECs).

### Experiment 2: iPSC Chips - 21 chips

- iPSC liver equivalent
- iPSC intestine equivalent
- iPSC neuronal equivalent with blood brain barrier
- iPSC renal equivalent

### Intestine compartment

The insert of the intestine equivalent contains organoids of iPSC-derived intestinal cells embedded into Matrigel containing also iPSC-derived fibroblasts and iPSC-derived endothelial cells. On the iPSC derived intestinal equivalent model, primary microvascular endothelial cells or iPSC-derived endothelial cells are seeded on the basolateral side of the 24-well standing cell culture insert.

### Neuronal compartment

The neuronal equivalent with blood brain barrier consists of a cell culture insert containing iPSC-derived neuronal spheroids enriched with iPSC-derived endothelial cells seeded on the basolateral side of the cell culture insert.

### Liver compartment

The liver equivalent is accomplished by placing spheroids of iPSC-derived hepatocytes with iPSC-derived fibroblasts into the respective cavity. 20 spheroids are placed onto one chip, each containing 50,000 cells (24:1 ratio of hepatocytes:fibroblasts).

### Kidney (renal) compartment

The kidney compartments are seeded with iPSC-derived renal cells.

### Results

The chips were homeostatic. This was shown by constant levels of glucose and LDH (Fig. 3). A constant glucose concentration suggested constant consumption and an active regulation of the available glucose. The glucose levels have reached equal levels in all compartments. Feeding only apically through the intestinal equivalent was sufficient to supply both, the "blood" and "urine" circuit. Also, LDH activity has been shown rather constant throughout the experiments. As compared to glucose, LDH levels are different in each compartment. The barrier function of the intestine equivalent and the renal equivalent is shown by different concentrations of LDH in all three compartments (i.e., blood circuit -> medium reservoir 1; urine circuit -> medium reservoir 2; and intestine compartment). The barriers, thus, differentiate between the crossing substances. The active or passive penetration of glucose, while retaining other substances, indicate practical interactions between subsets of the chip system.

The glomerular and tubular compartments were accomplished with a combination of a polycarbonate membrane and a tight layer of renal cells (Fig. 4). The former implements a technical barrier to filter big medium constituents and retards their diffusion. The latter reabsorbs substances and causes the build-up of concentration gradients across the "blood" and "urine" circuit. This gradient is visible not only for LDH but also for aspartate transaminase (AST, Fig. 5). Both their distinctly different concentrations demonstrate the function of the kidney membrane of the ADME-N chip. The AST can only originate from the liver equivalent as it is an intracellular enzyme found in hepatocytes and muscle cells.

All organ equivalents were immunohistochemically assessed to determine identity, functionality and viability (Fig. 6-9). The iPSC-derived intestine equivalent was formed by a layer of Matrigel covered with iPSC-derived fibroblasts. Intestinal organoids enclosing a lumen were incorporated into the gel (Fig. 6). The iPSC-derived intestinal cells expressed NaK-ATPase and Cyp3A4. In Experiment 1.2 a closed layer of primary microvascular endothelial cells was, further, forming another layer on the basolateral side of the cell culture insert.

The primary liver equivalent showed functional markers at the end of its two-week cultivation period (Fig. 7). Also, for the iPSC-derived liver equivalent functional markers were confirmed after three weeks of cultivation in the ADME-N chip (Fig. 8).

The iPSC-derived neuronal equivalent expressed functional markers for β-tubulin III at the end of its two-week cultivation period (Fig. 9). Endothelial cells printed in the hydrogel show vWF expression after two weeks of chip cultivation (Experiment 1.2). The tightness of the blood barrier was shown by TEER values of 20-78 Ω x cm² after two weeks of chip cultivation (Experiment 2).

### Experiment 3: Liver 3D equivalent with iPSC-derived hepatocytes, endothelial cells and fibroblasts.

Hepatocyte cell differentiation was carried out with modifications from Szkolnicka et al. (Szkolnicka *et al.,* 2014). Fibroblast differentiation was carried out with modifications from Zou *et al.,* 2013). Endothelial differentiation as performed after Harding et al. (Harding *et al.,* 2017). Spheroids were formed by using ultra-low attachment plates. Figure 13 shows immunofluorescence images after differentiation of iPSC-derived hepatocytes and 3D spheroid formation. Figure 14 shows transcription levels of selected hepatic marker genes in iPSC-derived liver equivalents (iPSC-derived hepatocytes). Transcription levels were analyzed by qPCR. The iPSC-derived liver equivalents were cultivated over two days to generate 3D liver spheroids including iPSC-derived hepatocytes, fibroblasts and endothelial cells. Data are normalized to the housekeeper TBP. Figure 15 shows the metabolization capacity of 3D liver equivalents to metabolize propranolol into propranolol-glucoronide, and alpha-naphthoxylactic acid, in single culture on a chip.

### Experiment 4: Intestinal equivalent consisting of iPSC-derived intestinal organoids, iPSC-derived fibroblast and iPSC-derived endothelial cells.

The intestinal model was built up solely from iPSC-derived cells from StemUse101 iPSCs (iPSC-derived intestinal organoids, iPSC-derived fibroblast and iPSC-derived endothelial cells). A high density of organoids was already seen after two weeks of co-culture of iPSC-derived fibroblast and organoids. After three weeks in static culture, the organoids fused together and grew in size. Visualization of iPSC-derived endothelial cells was possible under the insert edge. A monolayer of iPSC-derived endothelial cells was still visible after three weeks of static cultivation. Figure 16 shows the results.

### Experiment 5: Hydrogel barrier with iPSC-derived intestinal organoids, fibroblast and endothelia cells.

Hydrogels were bioprinted with fibroblasts and seeded with primary endothelial cells and intestinal organoids. Figure 17 shows the results.

### Experiment 6: iPSC-derived blood brain barrier in the chip.

Differentiation of iPSCs into cortical progenitor cell spheroids, referred to as neurospheres, was performed in a DASbox^{®} Mini Bioreactor System (Eppendorf). The culture protocol was adapted from Rigamonti *et al.,* 2016). Blood-brain endothelial cell differentiation was adapted from Lippmann *et al.,* 2014). Neurospheres were transferred into a Transwell and blood-brain endothelial cells were seeded underneath the membrane. Figure 18 shows the results.

### Experiment 7: ADME-N co-culture experiment. (ADME-N = ADME Chip plus neuronal equivalent)

In a proof of concept experiment, the combination of four different iPSC-derived organ models from the StemUse101 iPSC line was cultivated over two weeks in the ADME-N chip. The ADME-N chip is therefore also termed 4-Organ-Chip. The ADME-N chip was designed to host an intestinal standing 24-well insert model, liver equivalents, renal organoids and neurospheres in an arrangement emulating the human physiology. The intestinal barrier model was build up on a 24-well insert. Intestinal organoids were co-cultured with fibroblasts and a monolayer of iPSC-derived endothelial cells was seeded underneath the insert membrane. The liver model was assembled from iPSC-derived hepatocytes with iPSC-derived fibroblasts. A single-cell solution of renal organoids was used to seed the membrane in both kidney compartments six days prior to the start of the ADME-N co-culture. The neuronal tissue was differentiated in a bioreactor system and put as spheroids into a 96-well transwell. This combination of organs was chosen to allow for studies evaluating compound ADME profiles (= physiologically-based pharmacokinetic (PBPK) complied chip/profile). The complex setup of this experiment is shown in Figure 19 (cf. Experiment 2 and corresponding part of Fig. 2). Figure 20 shows an immunostaining characterization of the liver, intestinal, renal and neuronal model co-cultivated in the ADME-N chip for 14 days. Figure 21 shows qPCR results of the co-culture in the ADME-N chip of the iPSC-derived intestinal, liver, renal and brain model.

The four different iPSC-derived organ models were not only shown to be stable after two weeks of co-cultivation in the ADME-N chip, *i.e.,* maintained their phenotype. Surprisingly, it has been found that a four-organ chip with miniaturized human intestine, liver, brain and kidney equivalents, all of them pre-differentiated from iPSCs from the same donor, shows further differentiation of different iPSC-derived organ models over a 14-day cultivation period in a culture medium deprived of growth factors. This further differentiation is attributed to a cross-talk between the organ equivalents. Furthermore, an advanced maturation can be observed for different tissues over time. Significantly, it has been shown that different iPSC-derived organ models are stable during co-cultivation in the ADME-N (= ADME chip plus neuronal equivalent), *i.e.,* maintain their phenotype, and show a defined and consistent marker gene expression towards the directed tissue over time (Experiment 7 and Fig. 21). Intestine model (Fig. 21a): NaKATPase - sodium-potassium adenosine triphosphatase (nutrient transport in the small intestine); CDX2 - Caudal Type Homeobox 2 (important for transcriptional regulation of intestinal epithelium)

Liver model (Fig. 21b): Significant upregulated genes are Alb - Albumin (produced only in the liver); MPR2 - Multidrug resistance-associated protein 2 (MRP2) (expressed in the canalicular part of hepatocytes and is functional during the biliary transport), and AFP - Alpha-fetoprotein - fetal liver protein (maturation of hepatocytes).

Brain model (Fig. 21d): Significant upregulated genes are TBR1 - T-box, brain1 (transcription factor important for brain development), and MAPR2 - Microtubule-associated protein 2 (essential for neurogenesis).

The present disclosure establishes a successful systemic long-term co-cultivation of four different autologous iPSC-derived organ models from a single donor cultured in a physiologically based pharmacokinetic (PBPK) compliant microphysiological system (MPS), without the addition of extra growth factors.

### List of references (cited, inter alia, in Table 1):

- 1.: Szkolnicka, D., Farnworth, S. L., Lucendo-villarin, B. & Hay, D. C. Deriving Functional Hepatocytes from Pluripotent Stem Cells. 1-12 (2014). doi:10.1002/9780470151808.sc01g05s30
- 2.: Zou, L. et al. A simple method for deriving functional MSCs and applied for osteogenesis in 3D scaffolds. Sci. Rep. 3, 2243 (2013).
- 3.: Kauffman, A. L. et al., Directed differentiation protocols for successful human intestinal organoids derived from multiple induced pluripotent stem cell lines. 2, 1-10 (2015).
- 4.: Morizane, R. & Bonventre, J. V. Generation of nephron progenitor cells and kidney organoids from human pluripotent stem cells. Nat. Protoc. 12, 195-207 (2016).
- 5.: Rigamonti, A. et al. Stem Cell Reports. Stem Cell Reports 6, 993-1008 (2016).
- 6.: Lippmann Ethan S. et al. Human Blood-Brain Barrier Endothelial Cells Derived from Pluripotent Stem Cells. Nat. Biotechnol. 30, 783-791 (2012).
- 7.: Harding, A. et al. Highly Efficient Differentiation of Endothelial Cells from Pluripotent Stem Cells Requires the MAPK and the PI3K Pathways. Stem Cells 35, 909-919 (2017).

## Claims

1. A microfluidic device comprising:
(a) cell culture compartments, in which
(i) induced pluripotent stem cell (iPSC)-derived hepatocyte precursor cells;
(ii) iPSC-derived intestinal precursor cells;
(iii) iPSC-derived renal tubular precursor cells; and
(iv) iPSC-derived neuronal precursor cells,
are separately deposited;
wherein the iPSC-derived precursor cells according to (i), (ii), (iii), and (iv) are obtained from a single donor of iPSC obtained from a single type of somatic cell; and
(b) further comprising a reservoir compartment, which serves to add nutrient solution to the system.

2. A microfluidic device comprising:
(a) cell culture compartments, in which
(i) induced pluripotent stem cell (iPSC)-derived hepatocyte organoids;
(ii) iPSC-derived intestinal organoids; and
(iii) iPSC-derived renal tubular organoids;
are separately deposited;
wherein the iPSC-derived organoids according to (i), (ii), and (iii) are obtained from a single donor of iPSC obtained from a single type of somatic cell; and
(b) further comprising a reservoir compartment, which serves to add nutrient solution to the system.

3. The microfluidic device of any one of claims 1-2, wherein the microfluidic device comprises a first circuit and a second circuit, and wherein the cell culture compartments containing the iPSC-derived precursor cells according to (i), (ii), (iii), and (iv), or the iPSC-derived organoids according to (i), (ii), and (iii), respectively, form part of the first circuit, and wherein the second circuit comprising a filtration unit and a reabsorption unit, wherein both circuits are connected with each other via the filtration unit and the reabsorption unit.

4. The microfluidic device of claim 3, wherein the filtration unit comprises a filtration barrier that selectively allows the passage of molecules from the first circuit to the second circuit based on size and charge of the molecules.

5. Use of the microfluidic device of any one of claims 1-4 in analytical testing, diagnostics, research, target validation, toxicity studies, tissue engineering, tissue manufacturing, drug screening, and/or pharmacokinetic-pharmacodynamic analysis.

6. A method of detecting an analyte in a microphysiological system making use of the microfluidic device of any one of claims 1-4.

## Patentansprüche

1. Mikrofluidische Vorrichtung, umfassend:
(a) Zellkulturkompartimente, in denen
(i) von induzierten pluripotenten Stammzellen (iPSC) abgeleitete Hepatozyten-Vorläuferzellen;
(ii) von iPSC abgeleitete intestinale Vorläuferzellen;
(iii) von iPSC abgeleitete renale tubuläre Vorläuferzellen; und
(iv) von iPSC abgeleitete neuronale Vorläuferzellen,
separat eingebracht werden;
wobei die von iPSC abgeleiteten Vorläuferzellen gemäß (i), (ii), (iii) und (iv) von einem einzigen Donor von iPSC stammen, welcher aus einem einzigen Typ somatischer Zellen erhalten wurde;
und
(b) weiterhin umfassend ein Reservoir-Kompartiment, welches dazu dient, dem System Nährlösung zuzuführen.

2. Mikrofluidische Vorrichtung, umfassend:
(a) Zellkulturkompartimente, in denen
(i) von induzierten pluripotenten Stammzellen (iPSC) abgeleitete Hepatozyten-Organoide;
(ii) von iPSC abgeleitete intestinale Organoide; und
(iii) von iPSC abgeleitete renale tubuläre Organoide;
separat eingebracht werden;
wobei die von iPSC abgeleiteten Organoide gemäß (i), (ii) und (iii) von einem einzigen Donor von iPSC stammen, welcher aus einem einzigen Typ somatischer Zellen erhalten wurde;
und
(b) weiterhin umfassend ein Reservoir-Kompartiment, welches dazu dient, dem System Nährlösung zuzuführen.

3. Mikrofluidische Vorrichtung nach einem der Ansprüche 1-2, wobei die mikrofluidische Vorrichtung einen ersten Kreislauf und einen zweiten Kreislauf umfasst, und wobei die Zellkulturkompartimente, welche die von iPSC abgeleiteten Vorläuferzellen gemäß (i), (ii), (iii) und (iv), oder die von iPSC abgeleiteten Organoide gemäß (i), (ii) und (iii) enthalten, Teil des ersten Kreislaufs bilden, und wobei der zweite Kreislauf eine Filtrationseinheit und eine Reabsorptionseinheit umfasst, wobei beide Kreisläufe über die Filtrationseinheit und die Reabsorptionseinheit miteinander verbunden sind.

4. Mikrofluidische Vorrichtung nach Anspruch 3, wobei die Filtrationseinheit eine Filtrationsbarriere umfasst, welche selektiv den Durchgang von Molekülen vom ersten Kreislauf zum zweiten Kreislauf basierend auf der Größe und Ladung der Moleküle ermöglicht.

5. Verwendung der mikrofluidischen Vorrichtung nach einem der Ansprüche 1 bis 4 bei analytischen Untersuchungen, in der Diagnostik, in der Forschung, bei der Target-Validierung, bei Toxizitätsstudien, beim Tissue Engineering, beim Tissue Manufacturing, beim Arzneimittelscreening, und/oder bei der pharmakokinetischpharmakodynamischen Analyse.

6. Verfahren zum Nachweis eines Analyten in einem mikrophysiologischen System unter Verwendung der mikrofluidischen Vorrichtung nach einem der Ansprüche 1-4.

## Revendications

1. Dispositif microfluidique comprenant :
(a) des compartiments de culture cellulaire, dans lesquels
(i) des cellules précurseurs d'hépatocytes dérivées de cellules souches pluripotentes induites (iPSC) ;
(ii) des cellules précurseurs intestinales dérivées d'iPSC ;
(iii) des cellules précurseurs tubulaires rénales dérivées d'iPSC ; et
(iv) des cellules précurseurs neuronales dérivées d'iPSC,
sont déposées séparément ;
les cellules précurseurs dérivées d'iPSC selon (i), (ii), (iii) et (iv) étant obtenues à partir d'un donneur unique d'iPSC obtenues à partir d'un type unique de cellule somatique ; et
(b) comprenant en outre un compartiment réservoir, qui sert à ajouter de la solution nutritive au système.

2. Dispositif microfluidique comprenant :
(a) des compartiments de culture cellulaire, dans lesquels
(i) des organoïdes d'hépatocytes dérivés de cellules souches pluripotentes induites (iPSC) ;
(ii) des organoïdes intestinaux dérivés d'iPSC ; et
(iii) des organoïdes tubulaires rénaux dérivés d'iPSC ;
sont déposés séparément ;
les organoïdes dérivés d'iPSC selon (i), (ii) et (iii) étant obtenus à partir d'un donneur unique d'iPSC obtenues à partir d'un type unique de cellule somatique ; et
(b) comprenant en outre un compartiment réservoir, qui sert à ajouter de la solution nutritive au système.

3. Dispositif microfluidique selon l'une quelconque des revendications 1 à 2, le dispositif microfluidique comprenant un premier circuit et un second circuit, et les compartiments de culture cellulaire contenant respectivement les cellules précurseurs dérivées d'iPSC selon (i), (ii), (iii) et (iv), ou les organoïdes dérivés d'iPSC selon (i), (ii) et (iii) faisant partie du premier circuit, et le second circuit comprenant une unité de filtration et une unité de réabsorption, les deux circuits étant reliés l'un à l'autre par l'unité de filtration et l'unité de réabsorption.

4. Dispositif microfluidique selon la revendication 3, dans lequel l'unité de filtration comprend une barrière de filtration qui autorise sélectivement le passage de molécules du premier circuit au second circuit sur la base de la taille et de la charge des molécules.

5. Utilisation du dispositif microfluidique selon l'une quelconque des revendications 1 à 4 en essais analytiques, diagnostic, recherche, validation de cible, études de toxicité, ingénierie tissulaire, fabrication tissulaire, criblage de médicament et/ou analyse pharmacocinétique-pharmacologique.

6. Procédé de détection d'un analyte dans un système microphysiologique au moyen du dispositif microfluidique selon l'une quelconque des revendications 1 à 4.
